# NEW EUROPEAN PATENT SPECIFICATION

(11) **EP 0 211 148 B2**
(45) Date of publication and mention of the opposition decision: **24.11.1999**
(45) Mention of the grant of the patent: 26.08.1992
(21) Application number: 86105365.0
(22) Date of filing: 30.06.1981
(51) Int. Cl.: C12N 15/20, C12P 21/02, A61K 38/21, C07K 14/555, C12N 1/20

(54) **Mature human leukozyte interferons, process for their bacterial production, intermediates therefor and compositions containing them**
Reife menschliche Leukozyten-Interferone, Verfahren zu ihrer bacteriellen Herstellung, Zwischenprodukte hierfür und Zusammensetzungen diese enthaltend
Interferons humains matures d'origine leucocytaire, procédés pour leur péparation par l'utilisation de bactéries, produits intermédiaires dans la préparation desdits interferons et compositions contenant lesdits interferons

(30) Priority: 01.07.1980 US 164986; 08.09.1980 US 184909; 10.11.1980 US 205578; 21.04.1981 US 256204
(43) Date of publication of application: 25.02.1987
(62) Divisional of application: 81105067.3
(73) Proprietor: F. HOFFMANN-LA ROCHE AG, 4002 Basel (CH); GENENTECH, INC., South San Francisco California 94080 (US)
(72) Inventor: Goeddel, David Van Norman, Burlingame California (US); Pestka, Sidney, North Caldwell New Jersey (US)
(74) Representative: Lederer, Franz, Dr.

(56) References cited:
- EP-A- 0 018 218
- EP-A- 0 032 134
- EP-A- 0 034 307
- EP-A- 0 042 246
- EP-A- 0 051 873
- EP-A- 0 062 971
- FR-A- 2 442 054
- GB-A- 2 091 268
- NATURE, vol. 284, 27th March 1980, pages 316-320, Macmillan Journals Ltd.; S. NAGATA et al.: "Synthesis in E. coli of a polypeptide with human leukocyte interferon activity"
- GENE, vol. 10, no. 1, May 1980, pages 1-10, Elsevier/North-Holland Biomedical Press, NL; N. MANTEI et al.: "The nucleotide sequence of a cloned human leukocyte interferon cDNA"
- SCIENCE, vol. 209, 19th September 1980, pages 1343-1347, AAAS; M. STREULI et al.: "At least three human type alpha interferons: Structure of alpha 2"
- NATURE, vol. 281, 18th October 1979, pages 544-548, Macmillan Journals Ltd.; D.V. GOEDDEL et al.: "Direct expression in Escherichia coli of a DNA sequence coding for human growth hormone"
- NATURE, vol. 287, no. 5781, 2nd October 1980, pages 411-416, Macmillan Journals Ltd., Chesham, Bucks, GB; D.V. GOEDDEL et al.: "Human leukocyte interferon produced by E. coli is biologically active"
- NATURE, vol. 290, no. 5801, 5th March 1981, pages 20-26, Macmillan Journals Ltd., Chesham, Bucks, GB; D.V. GOEDDEL et al.: "The structure of eight distinct cloned human leukocyte interferon cDNAs"
- NUCLEIC ACIDS RESEARCH, vol. 9, no. 3, 1981, pages 731-741, IRL Press Ltd., London, GB; E. YELVERTON et al.: "Bacterial synthesis of a novel human leukocyte interferon"
- NATURE, vol. 287, 2nd October 1980, pages 401-408, Macmillan Journals Ltd.; S. NAGATA et al.: "The structure of one of the eight or more distinct chromosomal genes for human interferon-alpha"
- NATURE, vol. 287, 2nd October 1980, pages 408-411, Macmillan Journals Ltd.; G. ALLEN et al.: "A family of structural genes for human lymphoblastoid (leukocyte-type) interferon"
- RESEARCH DISCLOSURE, no. 183, July 1979, pages 361,362, article no. 18309; "The production of interferon by genetic engineering"

## Description

The present invention relates to the field of recombinant DNA technology, i.e. to processes used in recombinant DNA technology and to products obtained by these processes.

In a more detailed aspect the present invention relates to mature human bacterially produced leukocyte interferons, characterized in that they consist of 165-166 amino acids and contain Cys-Asp-Leu or in positions 1, 2 and 3 or it consists of 166 amino acids and contains Cys-Asn-Leu in positions 1, 2 and 3 and such mature leukocyte interferons with at the N-terminus an additional methionine residue, to pharmaceutical compositions containing them and to a process for their preparation which comprises causing a culture of a bacterium transformed with a replicable bacterial expression vehicle capable of expressing said polypeptides to grow up and express said polypeptides. The present invention also comprises the expression vehicles used in this process and the novel bacteria containing these expression vehicles as well as the processes for their preparation. Finally, the invention relates to DNA sequences comprising sequences coding for the amino acid sequence of a mature human leukocyte interferon.

### Background of the invention

Human leukocyte interferon (LelF) was first discovered and prepared in the form of very crude precipitates by Isaacs and Lindenmann (Proc. R. Soc. B 147,258-267 [1957]; U.S.P. 3.699.222). Efforts to purify and characterize the material have been ongoing since that time, and have led to the preparation of relatively homogeneous leukocyte interferons derived from normal or leukemic donors' leukocytes (German Offenlegungsschrift No. 2.947.134). These interferons are a family of proteins known to possess the ability to confer a virus-resistant state in their target cells. In addition. interferon can act to inhibit cell proliferation and modulate immune response. These properties have prompted the clinical use of leukocyte interferon as a therapeutic agent for the treatment of viral infections and malignancies.

Leukocyte interferons have been purified to essential homogeneity (Rubinstein et al., Proc. Natl. Acad. Sci. U.S.A. 76, 640-644 [1979]; Zoon et al., ibid. 76, 5601-5605 [1979]), and reported molecular weights range from about 17,500 to about 21,000. The specific activity of these preparations is remarkably high, 2 x 10⁸ to 1 x 10⁹ units/mg protein, but yields from cell culture methods have been discouragingly low. Nevertheless, advances in protein sequencing techniques have permitted the determination of partial amino acid sequences (Zoon et al., Science 207, 527 [1980]; Levy et al., Proc. Natl. Acad. Sci. U.S.A. 77, 5102-5104 [1980]). Elucidation of the glycosylation of various leukocyte interferons is not at present complete, but it is now clear that differences in glycosylation among family members does not alone account for the spectrum of molecular weights observed. Instead, the leukocyte interferons differ markedly in amino acid composition and sequence, and amino acid homology is. in some cases, less than 80 percent.

While isolation from donor leukocytes has provided sufficient material for partial characterization and limited clinical studies with homogeneous leukocyte interferon, it is a totally inadequate source for the amounts of interferon needed for large scale clinical tnals and for broad scale prophylactic and/or therapeutic use thereafter. Indeed, presently clinical investigations employing human leukocyte-derived interferons in antitumor and antiviral testing have principally been confined to crude (< 1 percent pure) preparations of the material, and long lead times for the manufacture of sufficient quantities, even at unrealistic price levels, have critically delayed investigation on an expanded front.

With the advent of recombinant DNA technology, however, the controlled microbial production of an enormous variety of useful polypeptides has become possible. Already in hand are bacteria modified by this technology to permit the production of such polypeptide products such as somatostatin, the A and B chains of human insulin and human growth hormone (Itakura et al., Science 198, 1056-1063 [1977]; Goeddel et al., Nature 281, 544-548 [1979]). More recently, recombinant DNA techniques have been used to occasion the bacterial production of proinsulin and thymosin alpha 1 and several authors have reported on the obtention of DNA coding for human leukocyte interferon and to resultant proteins having leukocyte interferon activcity (Nagata et al., Nature 284, 316-320 [1980]; Mantei et al., Gene 10, 1-10 [1980]; Taniguchi et al., Nature 285, 547-549 [1980]).

The workhorse of recombinant DNA technology is the plasmid, a non-chromosomal loop of doublestranded DNA found in bacteria and other microbes, oftentimes in multiple copies per cell. Included in the information encoded in the plasmid DNA is that required to reproduce the plasmid in daughter cells (i.e., a "replicon") and ordinarily, one or more selection characteristics such as, in the case of bacteria, resistance to antibiotics which permit clones of the host cell containing the plasmid of interest to be recognized and preferentially grown in selective media. The utility of plasmids lies in the fact that they can be specifically cleaved by one or another restriction endonuclease or "restriction enzyme", each of which recognizes a different site on the plasmidic DNA. Thereafter heterologous genes or gene fragments may be inserted into the plasmid by endwise joining at the cleavage site or at reconstructed ends adjacent to the cleavage site. DNA recombination is performed outside the cell, but the resulting "recombinant" plasmid can be introduced into it by a process known as transformation and large quantities of the heterologous gene-containing recombinant plasmid are obtained by growing the transformant. Moreover, where the gene is properly inserted with reference to portions of the plasmid which govern the transcription and translation of the encoded DNA message, the resulting expression vehicle can be used to actually produce the polypeptide sequence for which the inserted gene codes, a process referred to as expression.

Expression is initiated in a region known as the promoter which is recognized by and bound by RNA polymerase. In some cases, as in the tryptophan or "trp" promoter preferred in the practice of the present invention, promotor regions are overlapped by "operator" regions to form a combined promotor-operator. Operators are DNA sequences which are recognized by so-called repressor proteins which serve to regulate the frequency of transcription initiation at a particular promotor. The polymerase travels along the DNA, transcribing the information contained in the coding strands from its 5' to 3' end into messenger RNA which is in turn translated into a polypeptide having the amino acid sequence for which the DNA codes. Each amino acid is encoded by a nucleotide triplet or "codon" within what may for present purposes be referred to as the "structural gene", i.e. that part which encodes the amino acid sequence of the expressed product. After binding to the promoter, the RNA polymerase first transcribes nucleotides encoding a ribosome binding site, then a translation initiation or "start" signal (ordinarily ATG, which in the resulting messenger RNA becomes AUG), then the nucleotide codons within the structural gene itself. So-called stop codons are transcribed at the end of the structural gene whereafter the polymerase may form an additional sequence of messenger RNA which, because of the presence of the stop signal, will remain untranslated by the ribosomes. Ribosomes bind to the binding site provided on the messenger RNA, in bacteria ordinarily as the mRNA is being formed, and themselves produce the encoded polypeptide, beginning at the translation start signal and ending at the previously mentioned stop signal. The desired product is produced if the sequences encoding the ribosome binding site are positioned properly with respect to the AUG initiaton codon and if all remaining codons follow the initiaton codon in phase. The resulting product may be obtained by lysing the host cell and recovering the product by appropriate purification from other bacterial protein.

We perceived that application of recombinant DNA technology (i.e. the insertion of interferon genes in bacterial expression vehicles and their expression under the control of bacterial gene regulatory elements) would be the most effective way of providing large quantities of mature leukocyte interferon which, despite the absence in material so produced of the glycosylation characteristic of human-derived material, could be employed clinically in the treatment of a wide range of viral and neoplastic diseases.

The approach to obtaining a first leukocyte gene in a accordance with the present invention involved the following steps:
(1) Partial amino acid sequences of human leukocyte interferon purified to homogeneity were used to construct sets of synthetic DNA probes the codons of which, in the aggregate, represented all possible nucleotide combinations capable of encoding the partial amino acid sequences.
(2) Bacterial colony banks were prepared containing complementary DNA (cDNA) from induced messenger RNA. Other induced mRNA having been radio-labelled was hybridized to plasmid cDNA from this bank. Hybridizing mRNA was eluted and tested for translation into interferon in oocyte assay. Plasmid DNA from colonies shown to induce interferon activity in this manner have further been tested for hybridization to probes made as described in (1) above.
(3) Parallel to the approach in part (2) above, induced mRNA-derived cDNA in plasmids were used to form an independent bank of transformant colonies. The probes of part (1) were used to prime the synthesis of radio-labelled single stranded cDNA for use as hybridization probes. The synthetic probes hybridized with induced mRNA as template and were extended by reverse transcription to form induced, radio-labelled cDNA. Clones from the colony bank that hybridized to radio-labelled cDNA obtained in this manner have been investigated further to confirm the presence of a full-length interferon encoding gene. Any partial length putative gene fragment obtained in parts (1) or (2) can itself be used as a probe for the full-length gene.
(4) The full-length gene obtained above was tailored, using synthetic DNA, to eliminate any leader sequence that might prevent bacterial expression of the mature polypeptide and to permit appropriate positioning in an expression vehicle relative to start signals and the ribosome binding site of a bacterial promoter. Expressed interferon was purified to a point permitting confirmation of its character and determination of its activity.
(5) The interferon gene fragment prepared in the foregoing fashion was itself used in probing, by hybridization, for other partially homologous leukocyte interferon species.

In applying methods of recombinant DNA technology as outlined above the bacterial production in high yield and purity of the family of homologous leukocyte interferons (unglysosylated) as mature polypeptides, i.e. accompanied by the corresponding presequence or any portion thereof, was achieved. These interferons may be directly expressed, recovered and purified to levels fitting them for use in the treatment of viral and malignant diseases of animals and man. Family members so far expressed have proven efficacious in in vitro testing and, in the first such demonstration of its kind, in in vivo testing as well, the latter involving the first mature leukocyte interferon to have been bacterially produced.

The expression "mature leukocyte interferon" used in the context of the present application defines a bacterially produced leukocyte interferon molecule, devoid of glycosyl groups, which is immediately expressed from a translation start signal (ATG) just before the first amino acid codon of the natural product. The "mature" polypeptide may thus contain as the first amino acid in its sequence methionine (for which ATG codes) without essentially altering its character. On the other hand, the bacterial host may process the translation product to delete the initial methionine. "Expression" of mature leukocyte interferon connotes the bacterial production of an interferon molecule containing no glycosyl groups or a presequence that immediately attends mRNA translation of a human leukocyte interferon genome.

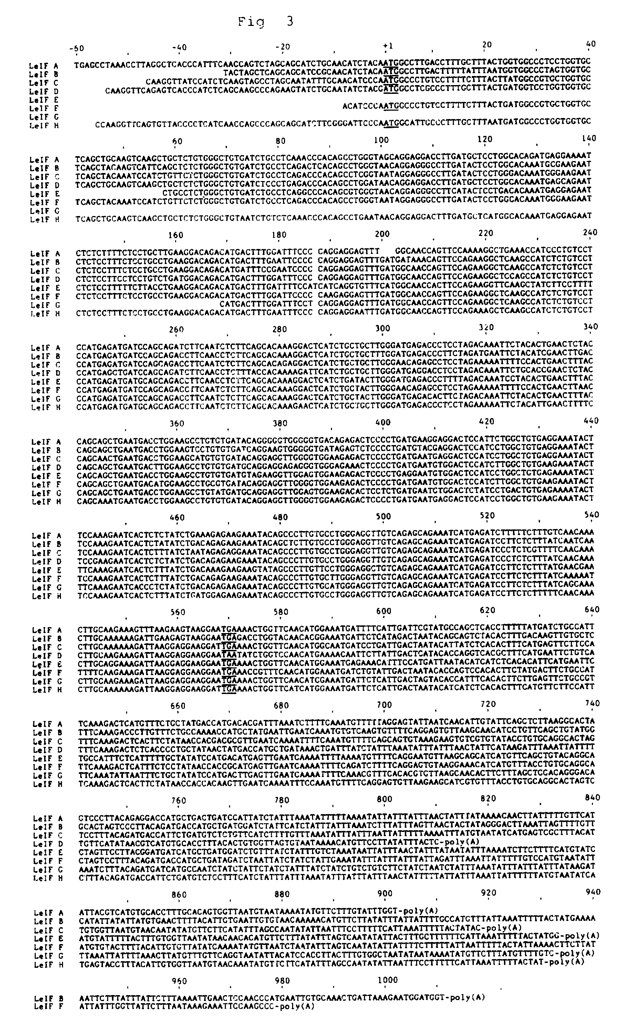

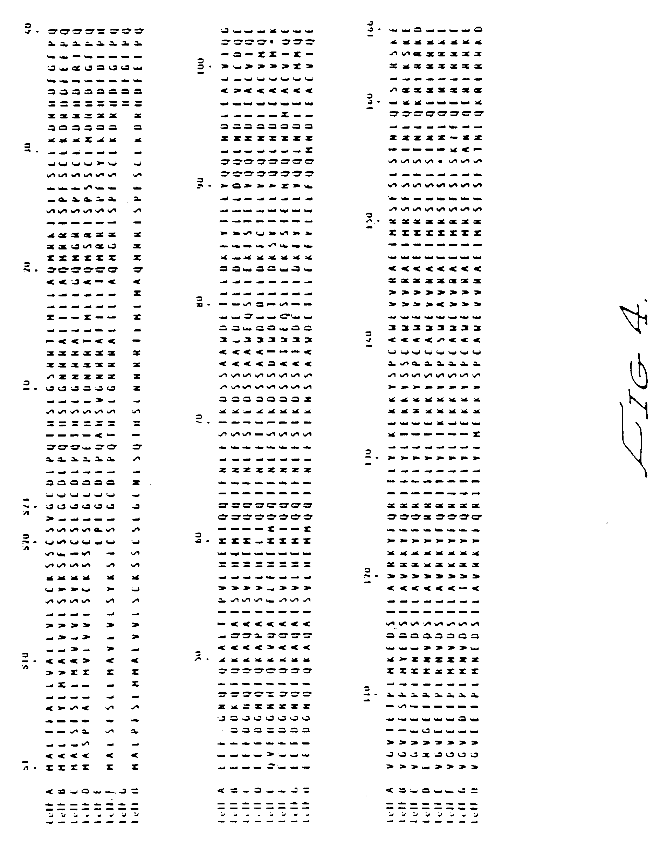

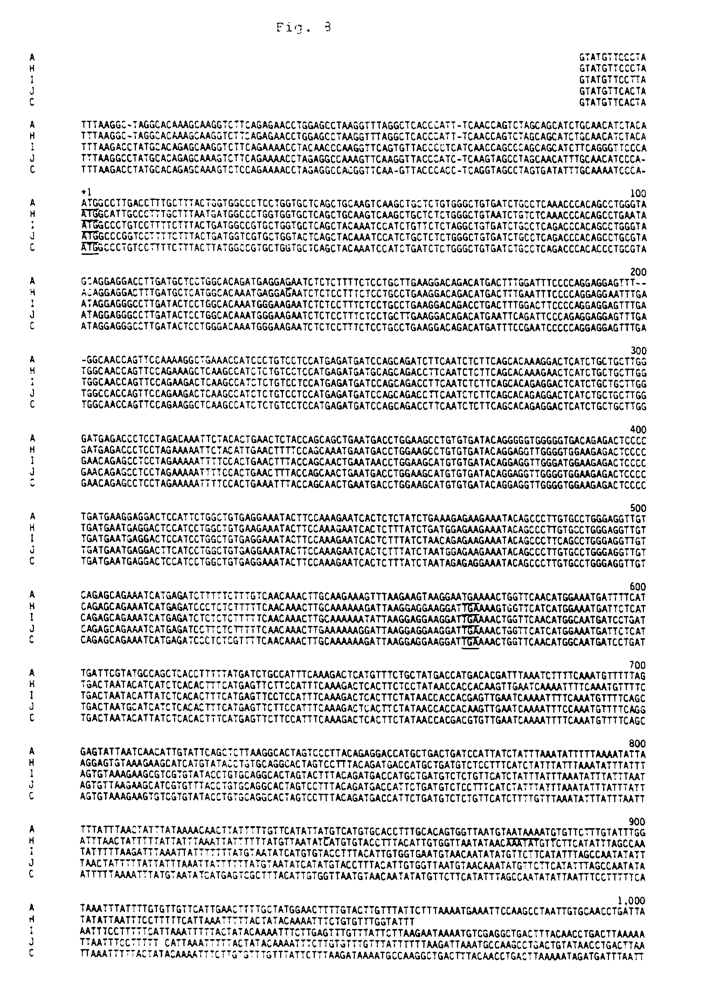

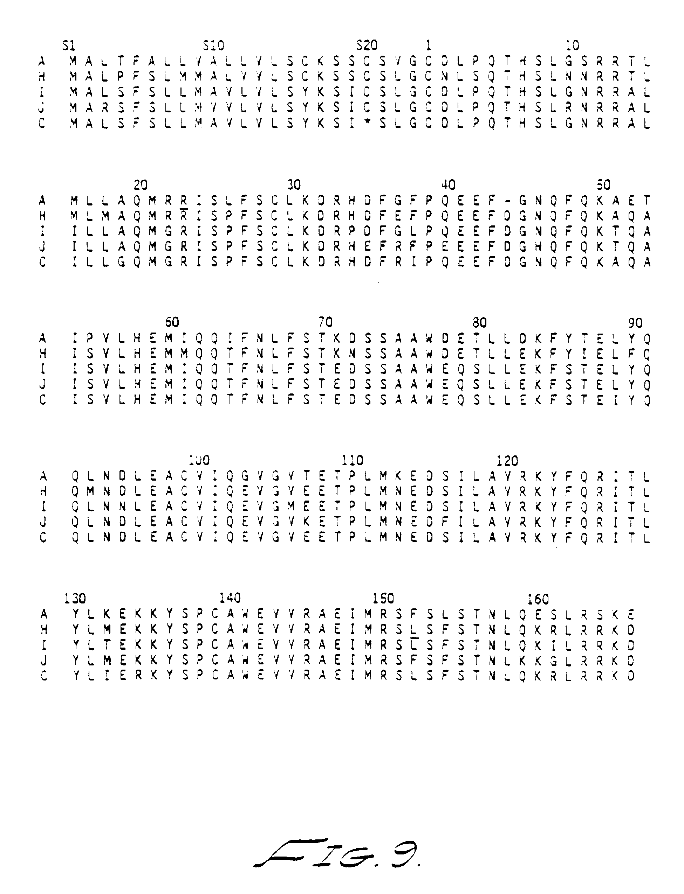

Particular mature leukocyte interferon proteins hereof have been defined by means of determined DNA gene (Figures 3 and 8) and deductive amino acid sequencing (Figures 4 and 9). It will be understood that for these particular interferons, indeed all of the family of leukocyte interferon proteins embraced herein, natural allelic variations exist and occur from individual to individual whereas the particular sequences I and J are not specifically claimed. These variations may be demonstrated by (an) amino acid difference(s) in the overall sequence or by deletions, substitutions, insertions, inversions or additions of(an) amino acid(s) in said sequence. For each mature leukocyte interferon protein hereof, labelled LelF A through LelF J such allelic variations are included within the scope of the label or term defining such, and thus, this invention.

Human leukocyte interferon A (LelF A), corresponding DNA, vectors, recombinant microorganisms as well as a process for its manufacture and its use in a pharmaceutical composition are claimed in the European Patent Application No. 81105067.3. The purpose of the references to LelF A in this specification is to provide a better understanding of the invention.

### Description of the drawings

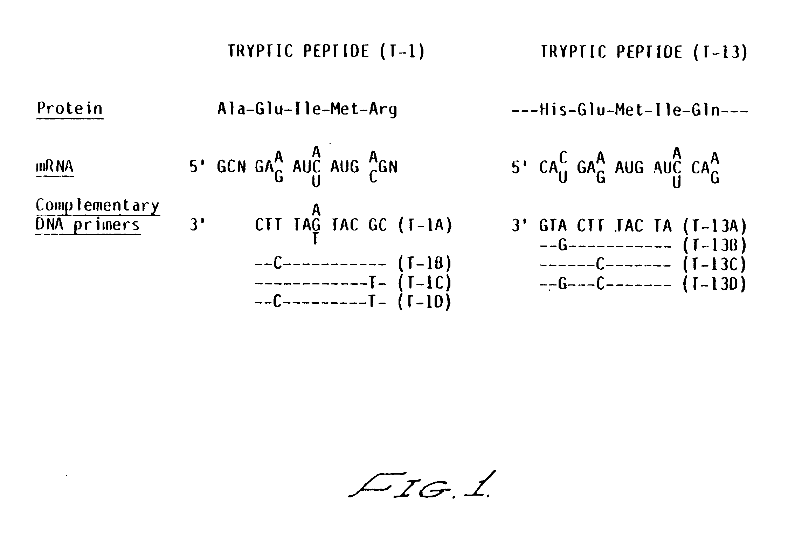

Figure 1 depicts two amino acid sequences which were common to all interferon species isolated from human leukocyte and purified to homogeneity designated T-1 and T-13. All potential mRNA sequences coding for these peptides are shown, as are the corresponding DNA sequences. The letters A, T, G, C and U respectively connote the nucleotides containing the bases adenine, thymine, guanine, cytosine and uracil. The letter N connotes any one of the nucleotides A, G, C and U. Polynucleotides are depicted as reading from the 5' (left) in the 3' (right) direction and. where double stranded ("d.s.") DNA is depicted, vice-versa for the bottom or non-coding strand.

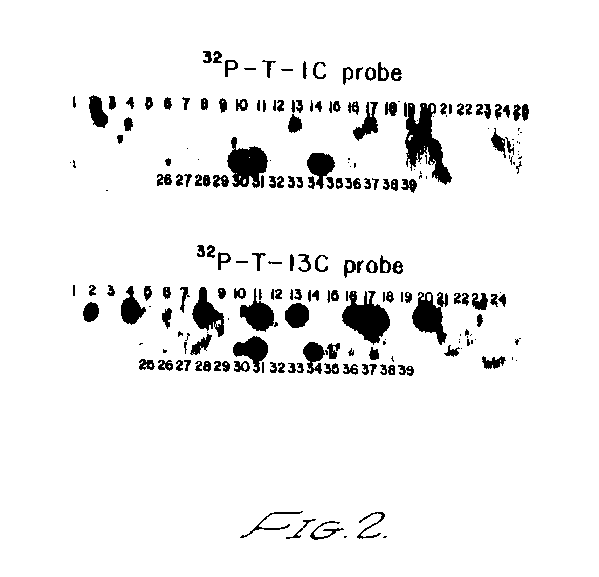

Figure 2 is an autoradiogram showing hybridization of potential LelF plasmids with ³²P-labelled synthetic deoxyoligonucleotides.

Figure 3 depicts the nucleotide sequences (coding strands) of eight gene fragments isolated as candidates for use in the expression of leukocyte interferons. respectively designated "A" through "H". The ATG translational initiation codon and the termination triplet for each LelF is underlined. The stop codons or termination triplets are followed by 3' untranslated regions. The included full-length gene for LelF A is missing one codon found in the others depicted, as indicated in the third A line of Figure 3. 5' untranslated regions precede the leader sequences. As isolated, fragment E lacks the full presequence of leader, but includes the entire gene for the putative mature LelF E. Fragment G as isolated lacks the full coding sequence.

Figure 4 is a comparison of the eight LelF protein sequences predicted from nucleotide sequences. The one letter abbreviations recommended by the IUPAC-IUB Commission on Biochemical Nomenclature are used: A, alanine; C, cysteine; D, aspartic acid; B, glutamic acid; F, phenylalanine; G, glycine; H, histidine; I, isoleucine; K, lysine; L, leucine; M, methionine; N, asparagine; P, proline; Q, glutamine; R, arginine; S. serine; T, threonine; V, valine; W. tryptophan; and Y, tyrosine. The numbers refer to amino acid positions (S refers to signal peptide). The dash in the 165 amino acid LelF A sequence at position 44 is introduced to align the LelF A sequence with the 166 amino acid sequences of the other LelFs. The LelF E sequence was determined by ignoring the extra nucleotide (position 187 of Figure 3) in its coding region. The asterisks indicate in-phase termination codons. The underlined residues are amino acids which are also present in human fibroblast interferon.

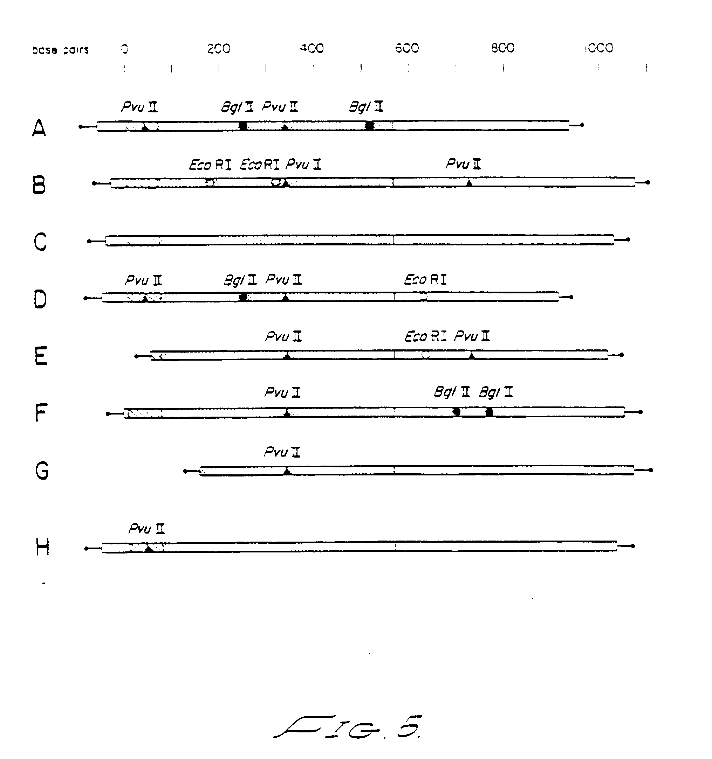

Figure 5 depicts restriction endonuclease maps of the eight types of LeIF cloned cDNAs (A through H). The hybrid plasmids were constructed by the dC:dG tailing method (Goeddel, D.V. et al, Nature 287, 411-416 [1980]). Therefor, the cDNA inserts can be exised using Pstl. The lines at the end of each cDNA insert represent the flanking homopolymeric dC:dG tails. The positions of PvuII, EcoRI and BgIII restriction sites are indicated. Shaded regions of the figure represent the coding sequences of mature LelFs; the crosshatched regions indicate signal peptide coding sequences; and the open regions show 3' and 5' non-coding sequences.

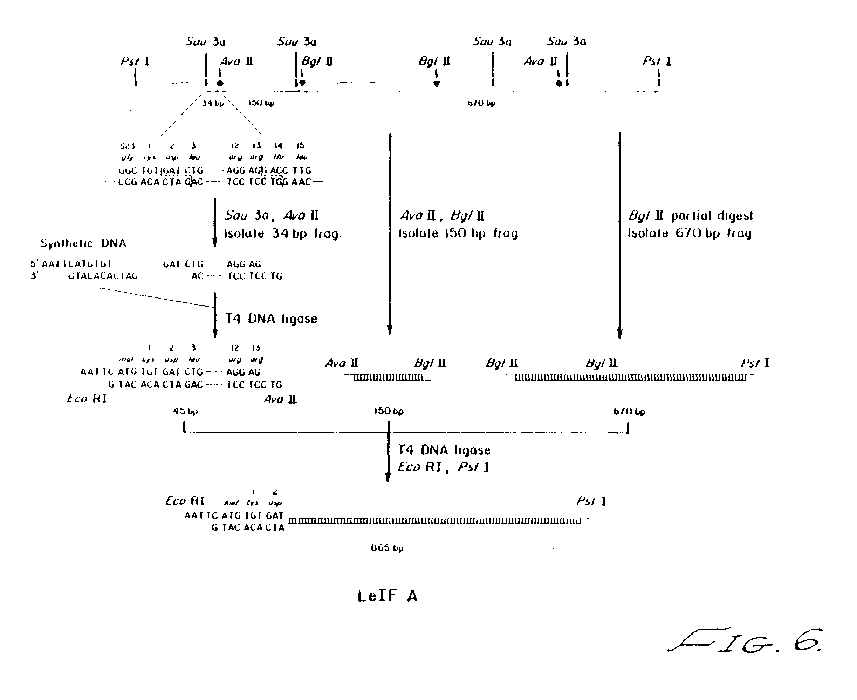

Figure 6 schematically depicts the construction of a gene coding for the direct bacterial synthesis of mature LelF A. Restriction sites and residues are as shown ("Pst I", etc.). The term "b.p." connotes "base pair".

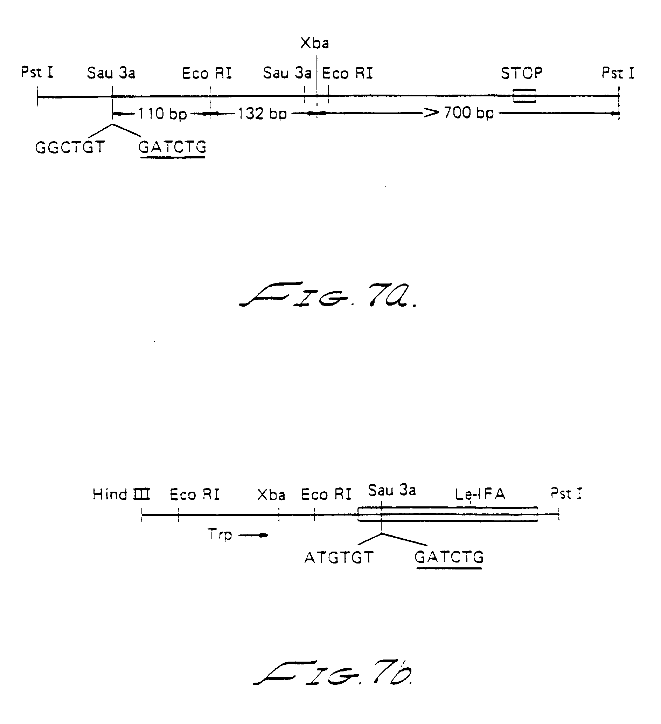

Figure 7 (not to scale) schematically depicts a restriction map of two gene fragments employed in expressing the mature leukocyte interferon LelF B. The codon sequences indicated are the coding strand termini resulting from digestion with the restriction enzyme Sau3a in the two cases shown.

Figures 8 and 9 provide the DNA and amino acid (see Figure 4 above for the corresponding one letter abbreviations) sequences of five LeIF proteins hereof, including types I and J, wherein the particular sequences I and J are not specifically claimed. In Figure 9, the asterisk indicates a termination codon in the corresponding DNA sequence and the hyphen a deletion or gap in the sequence.

### DESCRIPTION OF THE PREFERRED EMBODIMENTS

### A. Microorganisms employed

The work described involved use of two microorganisms: E. coli x 1776, as described in U.S.P. 4.190.495, and E. coli K-12 strain 294 (end A, thi⁻, hsr⁻, hsm⁺ₖ), as described in British patent publication No. 2055382 A. Each has been deposited with the American Type Culture Collection (ATCC accession Nos. 31537 and 31446 respectively). All recombinant DNA work was performed in compliance with applicable guidelines of the National Institutes of Health.

The invention, in its most preferred embodiments, is described with reference to E. coli, including not only strains E. coli x 1776 and E. coli K-12 strain 294, defined above, but also other known E. coli strains such as E. coli B, or other bacterial strains, many of which are deposited and available from recognized microorganism depository institutions, such as the American Type Culture Collection. See also German Offenlegungsschrift 2644432. These other microorganisms include, for example, Bacilli such as Bacillus subtilis and other enterobacteriaceae among with can be mentioned as examples Salmonella typhimurium and Serratia marcescens, utilizing plasmids that can replicate and express heterologous gene sequences therein.

### B. Source and purification of LeIF mRNA

LelF mRNA may be obtained from human leukocytes, ordinarily those of patients with chronic myelogenous leukemia, that have been induced to produce interferon with Sendai or Newcastle disease virus, as described in e.g. German Offenlegungsschrift No. 2947134. A particularly preferred source, and that used in the work reported herein, is a cell line designated KG-1 derived from a patient with acute myelogenous leukemia. The cell line, described by Koeffler, H.P. and Golde, D.W., Science 200, 1153 (1978), grows readily in a culture medium comprising RPMI (Rosewell Park Memorial Institute) 1640 plus 10% FCS (fetal calf serum) heat-inactivated, 25 mM HEPES buffer (N-2-hydroxy-ethyl-piperazine-N'-2-ethane-sulfonic acid) and 50 µg/ml of gentamicin, and is subcultured 1 to 3 split two times a week. Cells may be frozen from the foregoing growth medium plus 10% dimethylsulfoxide. KG-1 has been deposited with the American Type Culture Collection (ATCC accession No. CRL 8031).

KG-1 cells were induced to produce leukocyte interferon mRNA with Sendai or Newcastle disease virus following the procedure described by Rubinstein et al. (Proc. Natl. Acad. Sci. U.S.A. 76, 640-644 [1979]). Cells were harvested 5 hours after induction and RNA prepared by the guanidine thiocyanate-guanidine hydrochloride procedure (Chirgwin et al., Biochemistry 18, 5294-5299 [1979]). RNA from uninduced cells was isolated in the same manner. Oligo deoxythymidin (dT) - cellulose chromatography and sucrose gradient ultracentrifugation was used to obtain the 12S fraction of poly (A) mRNA as described by Green et al. (Arch. Biochem. Biophys. 172, 74-89 [1976]) and Okuyuma et al. (Arch. Biochem. Biophys. 188, 98-104 [1978]). This mRNA had an interferon titer of 8000-10.000 units per microgram in the Xenopus laevis oocyte assay (Cavalieri et al., Proc. Natl. Acad. Sci. U.S.A. 74, 3287-3291 [1977]).

### C. Preparation of colony banks containing LeIF cDNA sequences

5 µg of mRNA was used to prepare double stranded cDNA by standard procedures (Wickens et al., J. Biol. Chem. 253, 2483-2495 [1978] and Goeddel et al., Nature 281, 544-548 [1979]). The cDNA was size fractionated by electrophoresis on a 6% polyacrylamide gel and 230 ng of material ranging in size from 500 to 1500 b.p. were recovered by electroelution. A 100 ng portion of this cDNA was tailed with deoxycytidin (dC) residues as described by Chang et al., Nature 275, 617-624 (1978), annealed with 470 ng of plasmid pBR322 which has been tailed with deoxyguanosin (dG) residues at the PstI site (Bolivar et al., Gene 2, 95-113 [1977]), and used to transform E. coli x 1776. Approximately 130 tetracycline resistant, ampicillin sensitive transformants were obtained per ng of cDNA.

In a second similar experiment, approximately 1000 tetracycline resistant, ampicillin sensitive E. coli K-12 strain 294 transformants were obtained per ng of cDNA. In this case size fractionated cDNA material ranging in size from 600 to 1300 b.p. was recovered by electroelution for dC tailing.

### D. Preparation of synthetic oligonucleotides and their use

The knowledge of the amino acid sequences of several tryptic fragments of human leukocyte interferon permitted the design of synthetic deoxyoligonucleotides complementary to different regions of LelF mRNA. The two tryptic peptides T1 and T13 were selected because they had amino acid sequences requiring the synthesis of only 12 and 4 undecamers, respectively, to account for all possible coding sequences (Figure 1.). Four sets of deoxyoligonucleotide probes were synthesized for each sequence, containing either three (T-1A, B, C, D) or on (T-13A, B, C, D) oligonucleotide each. The indicated complementary deoxyoligonucleotides 11 bases long were chemically synthesized by the phosphotriester method (Crea et al., Proc. Natl. Acad. Sci. U.S.A. 75, 5765-5769 [1978]). Four individual probes were prepared in the T-13 series. The twelve T-1 probes were prepared in four pools of three probes as shown in Figure 1.

The four individual probes of the T-13 series and the twelve T-1 probes prepared in four pools of three primers each were used to prime the synthesis of radiolabelled single stranded cDNA for use as hybridization probes. The template mRNA was either the 12S RNA from Sendai-induced KG-1 cells (8000 units IF activity per µg) or total poly (A) mRNA from uninduced leukocytes (< 10 units per µg). ³²P-labelled cDNA was prepared from these primers using known reaction conditions (Noyes et al., Proc. Natl. Acad. Sci. U.S.A. 76, 1770-1774 [1979]). The 60 µl reactions were performed in 20 mM Tris-HCI (pH 8.3), 20mM KCI, 8mM MgCl₂, 30mM β-mercaptoethanol. Reactions included one µg of each primer (i.e. 12 µg total for T-1 series, 4µg total for T-13 series), 2µg of "induced" 12S fraction mRNA (or 10 µg of uninduced poly (A) mRNA), 0.5mM dATP, dCTP, dTTP, 200 µCi (α³² P) dCTP (Amersham, 2-3000 Ci/mmole), and 60 units reverse transcriptase (Bethesda Research Laboratories). Product was separated from unincorporated label by gel filtration on a 10 ml Sephadex® G-50 column, treated with 0.3N NaOH for 30 minutes at 70°C to destroy RNA, and neutralized with HCI. Hybridizations were performed as described by Kafatos et al., Nucleic Acids Res. 7, 1541-1552 (1979).

### E. Identification of clones pL1-pL30

The rapid plasmid isolation procedure of Birnboim et al., Nucleic Acids Res. 7, 1513-1523 (1979) was used to prepare 1 µg of plasmid DNA from each of 500 individual E. coli K-12 strain 294 transformants (see C). Each DNA sample was denatured and applied to nitrocellulose filters in triplicate following the procedure of Kafatos et al. (see above).

The three sets of nitrocellulose filters containing the 500 plasmid samples were hydridized with
a) induced cDNA primed with the T-1 set of primers,
b) T-13 primed induced cDNA, and
c) uninduced cDNA prepared by using both sets of primers. Clones were considered positive if they hybridized more strongly to one or both of the induced cDNA probes than to the total uninduced probe. Thirty "positive" clones (pL1-pL30) were selected from the 500 for further analysis.

### F. Identification of clones pL31-pL39

### Isolation of a plasmid (No. 104) containing a LeIF gene fragment

Transformants of E. coli x 1776 were screened by the colony hybridization procedure of Grunstein and Hogness (Proc. Natl. Acad. Sci. U.S.A. 72, 3961-3965 [1975]) using ³²P-labelled induced mRNA as probe (Lillenhaug et al., Biochemistry, 15, 1858-1865 [1976]). Unlabelled mRNA from uninduced cells was mixed with the probe at a ratio of 200 to 1 to compete with uninduced mRNA present in the ³²P-labelled preparation. Hybridization of labelled mRNA should occur preferentially to colonies containing induced sequences. Three classes of transformants were obtained:
(1) 2-3% of the colonies hybridized to ³²P-mRNA very strongly,
(2) 10% hybridized significantly less than class 1, and
(3) the remainder gave no detectable hybridization signal.

The positive colonies (classes (1) and (2) were examined for the presence of interferon-specific sequences by an assay which depends upon hybridization of interferon mRNA specifically to plasmid DNA. Initially, 60 strong positive colonies (class 1) were grown individually in 100 ml of M9 medium supplemented with tetracycline (20 µg/ml), diaminopimelic acid (100 µg/ml), thymidine (20 µg/ml), and d-biotin (1 µg/ml). The M9 medium contains per liter Na₂HPO₄ (6g), KH₂PO₄ (3 g), NaCl (0.5 g) and NH₄Cl (1 g). After autoclaving 1 ml of sterile 1M MgSO₄ and 10 ml of sterile 0.01 M CaCl₂ are added. Ten cultures were pooled and plasmid DNA was isolated from the six pools as described by Clewell et al., Biochemistry 9, 4428-440 [1970]. Ten µg of each plasmid DNA pool were cleaved with Hindlll, denatured and covalently bound to DBM (diazobenzyloxymethyl) paper. One µg of purified mRNA from induced cells was hybridized to each filter. Unhybridized mRNA was removed by washing. The specifically hybridized mRNA was eluted and translated in Xenopus laevis oocytes. By this assay, all six pools were negative. Five pools of ten colonies each and one pool of nine colonies were made from 59 weakly positive colonies, (class 2) and plasmids were prepared from the pools and examined as above. Among the six pools tested, one (K10) hybridized to interferon mRNA at levels significantly above background levels each time it was tested. In order to identify the specific interferon cDNA clone plasmid DNAs were prepared from the 9 colonies of pool K10 and examined individually. Two of the nine plasmids (No. 101 and No. 104) bound interferon mRNA well above background levels. From plasmid No. 104 a unique Bgl II restriction fragment containing 260 b.p. was isolated, labelled with ³²P using the procedure described by Taylor et al., Biochim. Biophys. Acta 442, 324-330 (1976), and used as probe to independently screen 400 E. coli 294 transformants by an in situ colony screening procedure (Grunstein and Hogness, Proc. Natl. Sci. U.S.A. 72, 3961-3965 [1975]). Nine colonies (pL31-pL39) were identified which hybridized to different extents with this probe.

In addition, the labelled 260 b.p. fragment was used to independently screen 4000 E. coli 294 transformants in the same manner. 50 colonies were identified which hybridized to different extents with this probe. One contained the LelF G fragment, one contained the LelF H fragment, and one contained a fragment designated LelF H1, an apparent allele of LelF H. The hybrid plasmids which result are designated "pLelF H", etc.

### G. Isolation and sequencing of a first full-length LeIf gene

Plasmid DNA was prepared from all 39 potential LelF cDNA clones and rescreened with the same 260 b.p. DNA probe using the hybridization procedure of Kafatos et al. (see above). Three plasmids (pL4, pL31, pL34) gave very strong hybridization signals, four (pL13, pL30, pL32, pL36) hybridized moderately, and three (pL6, pL8, pL14) hybridized weakly with the probe.

The 39 potential LelF cDNA recombinant plasmids were also screened by using ³²P-labelled synthetic undecamers (individual T-1 primer pools or individual T-13 primers) directly as hybridization probes. The hybridization conditions were chosen such that perfect base pairing should be required for detectable hybridization signals (Wallace et al., Nucleic Acids Res. 6, 3543-3557 [1979]). Thus, plasmid DNA from the 39 clones was prepared by a standard cleared lysate procedure (Clewell et al., see above) and purified by Biorad Agarose A-50 column chromatography. Samples (3 µg) of each preparation were linearized by treatment with Eco RI. denatured in alkali and spotted on 2 separate nitrocellulose filters, 1.5 µg per spot (Kafatos et al., see above). Individual synthetic deoxyoligonucleotide primers and primer pools were phosphorylated with (γ³² P)ATP as follows: 50 pmoles of oligonucleotide and 100 pmoles of (γ³²P)ATP (New England Nuclear, 2500 Ci/mmole) were combined in 30 µl of 50mM Tris-HCl, 10 mM MgCl₂ 15 mM β-mercaptoethanol. 2 units of T4 polynucleotide kinase were added and, after 30 minutes at 37°C, ³²P-labelled primers were purified by chromatography on 10 ml Sephadex® G-50 columns. Hybridizations were performed using 10⁶ cpm or primer T-13C or 3x10⁶ cpm of primer pool T-1C. The hybridizations were performed at 15°C for 14 hours in 6 x SSC [1 x SSC = 0.15 M NaCI, 0,015 M sodium citrate, pH 7.2], 10 x Denhardt's [0,2% bovine serum albumine, 0,2% polyvinylpyrolidone, 0.2% Ficoll] solution, as described by Wallace et al. (see above). Filters were washed for 5 minutes (3 times) at 0°C in 6 x SSC, dried, and exposed to x-ray film. Results are shown in Fig. 2 for ³²P-primer pool T-13C and primer T-1C.

Plasmid DNA from clone 104 was found to give significant hybridization with primer pool T-1C and primer T-13C, but no detectable hybridization with the other undecamers. As shown in Figure 2, several of the 39 potential LelF plasmids (pL2, 4, 13, 17, 20, 30, 31, 34) also hybridized with both of these probes. However, restriction analysis showed that only one of these plasmids, pL31, also contained a 260 b.p. internal BgIII fragment. PstI digestion of pL31 showed the size of the cDNA insert to be approximately 1000 b.p.

The entire PstI insert of pL31 was sequenced by both the Maxam-Gilbert chemical method (Methods Enzymol. 65, 499-560 [1980]) and by the dideoxy chain termination procedure (Smith, Methods Enzymol. 65, 560-580 [1980]) after subcloning Sau3a fragments into an M13 vector. The DNA sequence is shown ("A") in Figure 3. The appropriate translational reading frame could be predicted from protein sequence information in hand, the known range of LelF molecular weights, and the relative incidence of stop triplets in the three possible reading frames, and that in turn permitted prediction of the entire LelF amino acid sequence, including a pre- or signal peptide. The first ATG translational initiation codon is found 60 nucelotides from the 5' end of the sequence and is followed, 188 codons later, by a TGA termination triplet; there are 342 untranslated nucleotides at the 3' end, followed by a poly (A) sequence. The putative signal peptide (presumably involved in the secretion of mature LelF from leukocytes) is 23 amino acids long. The 165 amino acids constituting the mature LelF have a calculated MW of 19,390. We have termed the LeIf encoded by pL31 "LeIF A." It can be seen from the sequence data ("A") in Fig. 4 that the tryptic peptides T1 and T13 of LeIF B correspond to amino acids 145-149 and 57-61 respectively of LelF A. The actual DNA coding sequences found in these two regions are those represented by primer pool T1-C and primer T13-C (see Fig. 8)

### H. Direct expression of mature leukocyte interferon A (LeIF A)

### 1. Generally

The procedure followed to express LeIF A directly as a mature interferon polypeptide is a variant of that earlier employed for human growth hormone (Goeddel et al., Nature 281, 544-548 [1979]), insofar as it involved the combination of synthetic (N-terminal) and complementary DNAs.

As shown in Figure 6, a Sau3a restriction endonuclease site is conveniently located between codons 1 and 3 of LelF A. Two synthetic deoxyoligonucleotides were designed which incorporate an ATG translational initiation codon, restore the codon for amino acid 1 (cysteine), and create an EcoRI sticky end. These oligomers were ligated to a 34 b.p. Sau3a - Avall fragment of pL31. The resulting 45 b.p. product was ligated to two additional DNA fragments to construct an 865 b.p. synthetic-natural hybrid gene which codes for LelF A and which is bounded by EcoRI and PstI restriction sites. This gene was inserted into pBR322 between the EcoRI and PstI sites to give the plasmid pLeIF A1.

### 2. Construction of the tryptophan control element (containing the E. coli trp promoter, operator and trp leader ribosome binding site but lacking an ATG sequence for initiation of translation).

Plasmid pGMI carries the E. coli tryptophan operon containing the deletion ΔLE1413 (Miozzari et al., J. Bacteriology 133, 1457-1466 [1978]) and hence expresses a fusion protein comprising the first 6 amino acids of the trp leader and approximately the last third of the trp E polypeptide (hereinafter referred to in conjunction as LE'), as well as the trp D polypeptide in its entirety, all under the control of the trp promoter-operator system. The plasmid, 20 µg, was digested with the restriction enzyme PvuII which cleaves the plasmid at five sites. The gene fragments were next combined with EcoRI linkers (consisting of a self complementary oligonucleotide of the sequence: pCATGAATTCATG) providing an EcoRI cleavage site for a later cloning into a plasmid containing an EcoRI site. The 20 µg of DNA fragments obtained from pGM1 were treated with 10 units T₄ DNA ligase in the presence of 200 pmoles of the 5'-phosphorylated synthetic oligonucleotide pCATGAATTCATG and in 20 ul T₄ DNA ligase buffer (20mM Tris, pH 7.6, 0.5 mM ATP, 10 mM MgCl₂, 5 mM dithiothreitol) at 4°C overnight. The solution was then heated 10 minutes at 70°C to halt ligation. The linkers were cleaved by EcoRI digestion and the fragments, now with EcoRI ends were separated using 5 percent polyacrylamide gel electrophoresis (hereinafter PAGE) and the three largest fragments isolated from the gel by first staining with ethidium bromide, locating the fragments with ultraviolet light, and cutting from the gel the portions of interest. Each gel fragment, with 300 microliters 0.1 x TBE, was placed in a dialysis bag and subjected to electrophoresis at 100 V for one hour in 0.1 x TBE buffer (TBE buffer contains: 10.8 g Tris base, 5.5 g boric acid, 0.09 g Na₂EDTA in 1 liter H₂O). The aqueous solution was collected from the dialysis bag, phenol extracted, chloroform extracted and made 0.2 M sodium chloride, and the DNA recovered in water after ethanol precipitation. The trp promoter-operator-containing gene with EcoRI sticky ends was identified in the procedure next described, which entails the insertion of fragments into a tetracycline sensitive plasmid which, upon promoter-operator insertion, becomes tetracycline resistant.

Plasmid pBRHI (Rodriguez et al., Nucleic Acids Res. 6, 3267-3287 [1979]) expresses ampicillin resistance and contains the gene for tetracycline resistance but, there being no associated promoter, does not express that resistance. The plasmid is accordingly tetracycline sensitive. By introducing a promoter-operator system in the EcoRI site, the plasmid can be made tetracycline resistant.

pBRH1 was digested with EcoRI and the enzyme removed by phenol extraction followed by chloroform extraction and recovered in water after ethanol precipitation. The resulting DNA molecule was, in separate reaction mixtures, combined with each of the three DNA fragments obtained above and ligated with T₄ DNA ligase as previously described. The DNA present in the reaction mixture was used to transform competent E. coli K-12 strain 294 by standard techniques (Hershfield et al., Proc. Natl. Acad. Sci. U.S.A. 71, 3455-3459 [1974]) and the bacteria plated on LB (Luria-Bertani) plates containing 20 µg/ml ampicillin and 5 µg/ml tetracycline. Several tetracycline-resistant colonies were selected, plasmid DNA isolated and the presence of the desired fragment confirmed by restriction enzyme analysis. The resulting plasmid is designated pBRHtrp.

An EcoRI and BamHI digestion product of the viral genome of hepatitis B was obtained by conventional means and cloned into the EcoRI and BamHI sites of plasmid pGH6 (Goeddel et al, Nature 281, 544 [1979]) to form the plasmid pHS32. Plasmid pHS32 was cleaved with Xbal, phenol extracted, chloroform extracted and ethanol precipitated. It was then treated with 1 µl E. coli DNA polymerase I, Klenow fragment (Boehringer-Mannheim) in 30 µl polymerase buffer (50 mM potassium phosphate pH 7.4, 7mM MgCl₂, 1 mM β-mercaptoethanol) containing 0.1 mM dTTP and 0.1mM dCTP for 30 minutes at 0°C then 2 hours at 37°C. This treatment causes 2 of the 4 nucleotides complementary to the 5' protruding end of the Xbal cleavage site to be filled in: 
5'          CTAGA ——          5'          CTAGA ——
3'              T ——          3'            TCT ——
 Two nucleotides, dC and dT, were incorporated giving an end with two 5' protruding nucleotides. This linear residue of plasmid pHS32 (after phenol and chloroform extraction and recovery in water after ethanol precipitation) was cleaved with EcoRI. The large plasmid fragment was separated from the smaller EcoRI-XbaI fragment by PAGE and isolated after electroelution. This DNA fragment from pHS32 (0.2 µg), was ligated, under conditions similar to those described above, to the EcoRI-Taql fragment of the tryptophan operon (∼ 0.01 µg), derived from pBRHtrp.

In the process of ligating the fragment from pHS32 to the EcoRI - Taql fragment, as described above, the Taql protruding end is ligated to the Xbal remaining protruding end even though it is not completely Watson-Crick base-paired:

— T          CTAGA —          — TCTAGA —
— AGC    +     TCT —    ——▷   — AGCTCT —

A portion of this ligation reaction mixture was transformed into E. coli 294 cells, heat treated and plated on LB plates containing ampicillin. Twenty-four colonies were selected, grown in 3 ml LB (Luria-Bertani) media, and plasmid isolated. Six of these were found to have the Xbal site regenerated via E. coli catalyzed DNA repair and replication: 
— TCTAGA —          — TCTAGA —
                 →
— AGCTCT —          — AGATCT —
 These plasmids were also found to cleave both with EcoRI and Hpal and to give the expected restriction fragments. One plasmid, designated pTrp14, was used for expression of heterologous polypeptides, as next discussed.

The plasmid pHGH 107 (Goeddel et al., Nature 281, 544, [1979]) contains a gene for human growth hormone made up of 23 amino acid codons produced from synthetic DNA fragments and 163 amino acid codons obtained from complementary DNA produced via reverse transcription of human growth hormone messenger RNA. This gene, though it lacks the codons of the "pre" sequence of human growth hormone, does contain an ATG translation initiation codon. The gene was isolated from 10 µg pHGH 107 after treatment with EcoRI followed by E coli DNA polymerase I Klenow fragment and dTTP and dATP as described above. Following phenol and chloroform extraction and ethanol precipitation the plasmid was treated with BamHI.

The human growth hormone (HGH) gene-containing fragment was isolated by PAGE followed by electroelution. The resulting DNA fragment also contains the first 350 nucleotides of the tetracycline resistance structural gene, but lacks the tetracycline promoter-operator system so that, when subsequently cloned into an expression plasmid, plasmids containing the insert can be located by the restoration of tetracycline resistance. Because the EcoRI end of the fragment has been filled in by the Klenow polymerase I procedure, the fragment has one blunt and one sticky end, ensuring proper orientation when later inserted into an expression plasmid.

The expression plasmid pTrp14 was next prepared to receive the HGH gene-containing fragment prepared above. Thus, pTrp14 was Xbal digested and the resulting sticky ends filled in with the Klenow polymerase I procedure employing dATP, dTTP, dGTP and dCTP. After phenol and chloroform extraction and ethanol precipitation the resulting DNA was treated with BamHI and the resulting large plasmid fragment isolated by PAGE and electroelution. The pTrp14-derived fragment had one blunt and one sticky end, permitting recombination in proper orientation with the HGH gene containing fragment previously described.

The HGH gene fragment and the pTrp14 ΔXba-BamHI fragment were combined and ligated together under conditions similar to those described above. The filled in Xbal and EcoRI ends ligated together by blunt end ligation to recreate both the Xbal and the EcoRI site: 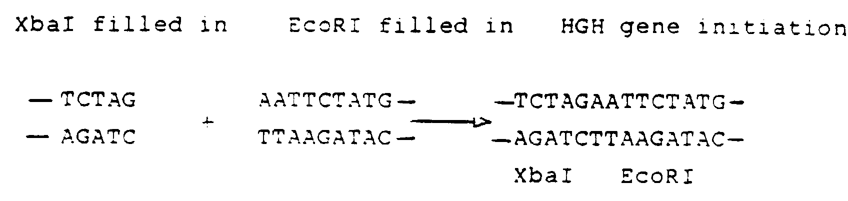
XbaI filled in    EcoRI filled in    HGH gene initiation

— TCTAG    AATTCTATG —    —TCTAGAATTCTATG—
                +                   
— AGATC    TTAAGATAC —    —AGATCTTAAGATAC—

XbaI    EcoRI
 This construction also recreates the tetracycline resistance gene. Since the plasmid pHGH 107 expresses tetracycline resistance from a promoter lying upstream from the HGH gene (the lac promoter), this construction designated pHGH 207, permits expression of the gene for tetracycline resistance under the control of the tryptophan promoter-operator. Thus the ligation mixture was transformed into E. coli 294 and colonies selected on LB plates containing 5 µg/ ml tetracycline.

Plasmid pHGH 207 was EcoRI digested and a 300 b.p. fragment containing the trp promoter, operator and trp leader ribosone binding site but lacking an ATG sequence for initiation of translation was recovered by PAGE followed by electroelution. This DNA fragment was cloned into the EcoRI site of pLelF A. Expression plasmids containing the above modified trp regulon (E. coli trp operon from which the attenuator sequence has been deleted to controllably heighten expression levels) can be grown to predetermined levels in nutrient media containing additive tryptophan in quantities sufficient to repress the promoter-operator system, then be deprived of tryptophan so as to derepress the system and occasion the expression of the intended product.

More particularly, and with reference to Figure 6, 250 µg of plasmid pL31 were digested with PstI and the 1000 b.p. insert isolated by gel electrophoresis on a 6% polyacrylamide gel. Approximately 40 µg of insert was electroeluted from the gel and divided into 3 aliquots for further digestion: a) A 16 µg sample of this fragment was partially digested with 40 units of BgIII for 45 minutes at 37°C and the reaction mixture purified on a 6% polyacrylamide gel. Approximately 2 µg of the desired 670 b.p. fragment were recovered. b) Another sample (8 µg) of the 1000 b.p. PstI insert was restricted with Avall and Bglll. One µg of the indicated 150 b.p. fragment was recovered after gel electrophoresis. c) 16 µg of the 1000 b.p. piece was treated with Sau3a and Avall. After electrophoresis on a 10% polyacrylamide gel, approximately 0.25 µg (10 pmole) of the 34 b.p. fragment was recovered. The two indicated deoxyoligonucleotides, 5'-dAAT-TCATGTGT (fragment 1) and 5'-dGATCACACATG (fragment 2) were synthesized by the phosphotriester procedure. Fragment 2 was phosphorylated as follows. 200 µl (∼ 40 pmole) of (γ³²P) ATP (Amersham, 5000 Ci/mmole) was dried down and resuspended in 30 µl of 60 mM Tris-HCI (pH8), 10mM MgCl₂, 15 mM β-mercaptoethanol, containing 100 pmoles of DNA fragment and 2 units of T4 polynucleotide kinase. After 15 minutes at 37°C, 1 µl of 10mM ATP was added and the reaction allowed to proceed another 15 minutes. The mixture was then heated at 70°C for 15 minutes, combined with 100 pmole of 5'-OH fragment 1 and 10 pmole of the 34 b.p. Sau3a - Avall fragment. Ligation was performed for 5 hours at 4°C in 50 µl of 20mM Tris-HCI (pH7.5) 10mM Mg Cl₂, 10 mM dithiothreitol, 0.5mM ATP and 10 units T4 DNA ligase. The mixture was electrophoresed on a 6% polyacrylamide gel and the 45 b.p. product recovered by electroelution. About 30 ng (1 pmole) of the 45 b.p. product were combined with 0.5 µg (5 pmoles) of the 150 b.p. Avall - Bglll fragment and 1 µg (2 pmoles) of the 670 b.p. BgIII - PstI fragment. The ligation was performed at 20°C for 16 hours using 20 units of T4 DNA ligase. The ligase was inactivated by heating to 65°C for 10 minutes. The mixture was then digested with EcoRI and PstI to eliminate polymers of the gene. The mixture was purified by 6% PAGE. About 20 ng (0.04 pmole) of the 865 b.p. product were isolated. One-half (10ng) of this was ligated into pBR322 (0.3 µg) between the EcoRI and PstI sites. Transformation of E. coli 294 gave 70 tetracycline resistant, ampicillin sensitive transformants. Plasmid DNA isolated from 18 of these transformants was digested with EcoRI and Pstl. 16 of the 18 plasmids had an EcoRI - Pstl fragment 865 b.p. in length. One µg of one of these, pLelF A1, was digested with EcoRI and ligated to the 300 b.p. EcoRI fragment (0.1 µg) containing the E. coli trp promoter and trp leader ribosome binding site, prepared as described above. Transformants containing the trp promoter were identified using a ³²P-trp probe in conjunction with the Grunstein-Hogness colony screening procedure. An asymetrically located Xbal site in the trp fragment allowed determination of recombinants in which the trp promoter was oriented in the direction of the LelF A gene.

### I. In vitro and in vivo activity of LeIF A

Extracts were prepared for IF assay as follows: one ml cultures were grown in L broth containing 5 µg/ml tetracycline to an A₅₅₀ value of about 1.0, then diluted into 25 ml of M9 media containing 5 µg/ml tetracycline. 10 ml samples were harvested by centrifugation when A₅₅₀ reached 1.0 and cell pellets were suspended in 1 ml of 15 percent sucrose, 50 mM Tris-HCI (pH 8.0), 50 mM EDTA. One mg of lysozyme was added and, after 5 minutes at 0°C, cells were disrupted by sonication. The samples were centrifuged 10 minutes (15,000 rpm) and interferon activity in the supernatants was determined by comparison with LeIF standards by the cytopathic effect (CPE) inhibition assay. To determine the number of IF molecules per cell a LeIF specific activity of 4 x 10⁸ units/mg was used.

As shown in Table 1, Clone pLelF A trp 25, in which the trp promoter was inserted in the desired orientation, gives high levels of activity (as high as 2.5 x 10⁸ units per liter). As shown in Table 2, the IF produced by E. coli K-12 strain 294/pLelF A trp 25 behaves like authentic human LelF; it is stable to treatment at pH 2 and is neutralized by rabbit anti-human leukocyte antibodies. The interferon has an apparent molecular weight of approximately 20,000.

The in vivo efficacy of interferon requires the presence of macrophages and natural killer (NK) cells and the in vivo mode of action appears to involve stimulation of these cells. Thus, it remained possible that the interferon produced by E. coli 294/pLelF A 25, while having antiviral activity in the cell culture assay, would not be active in infected animals. Moreover, the in vivo antiviral activity of the bacterially produced, non-glycosylated LelF A might be different from the glycosylated LelF derived from human "buffy coat" leukocytes. Therefore the biological activity of bacterially synthesized LelF A (2% pure) was compared with buffy coat LelF (8% pure) in lethal encephalomyocarditis (EMC) virus infection of squirrel monkeys (Table 3).

**TABLE 1**

| Interferon activity in extracts of E. coli | | | |
|---|---|---|---|
| E. coli K-12 strain 294 transformed by | Cell density (cells/ml) | IF Activity units/ml culture | LeIF molecules per cell |
| pLeIF A trp 25 | 3.5 x 10⁸ | 36,000 | 9,000 |
| pLeIF A trp 25 | 1.8 x 10⁹ | 250,000 | 12,000 |

**TABLE 2**

| Comparison of activities of extracts from E. coli 294/pLeIF A 25 with standard LeIF* | | | |
|---|---|---|---|
| | Interferon Activity (units/ml) | | |
| | untreated | pH 2 | rabbit anti-human leukocyte antibodies |
| 294/pLeIF A trp 25 extract | 500 | 500 | < 10 |
| LeIF standard | 500 | 500 | < 10 |

| | | | |
|---|---|---|---|
| * The 250,000 units/ml extract of E. coli 294/pLeIF A trp 25 described in Table 1 was diluted 500-fold with minimal essential medium giving a specific activity of 500 units/ml. A leukocyte interferon standard (Wadley Institute) previously titrated against the NIH leukocyte interferon standard, was also diluted to a final concentration of 500 U/ml. One ml aliquots were adjusted to pH 2 with 1N HCl, incubated at 4°C for 52 hours, neutralized by addition of NaOH and IF activity determined by the standard CPE inhibition assay. 25 µl aliquots of the 500 units/ml samples (untreated) were incubated with 25 µl of rabbit anti-human leukocyte interferon for 60 minutes at 37°C, centrifuged at 12,000 x g for 5 minutes and the supernatant assayed. | | | |

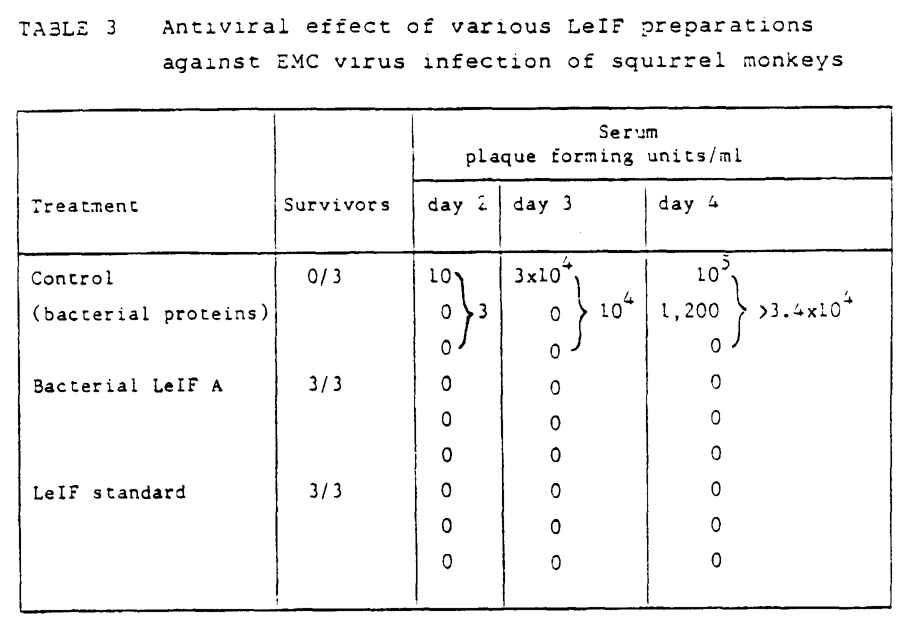

All monkeys were male (average weight 713 g) and had no EMC virus antibodies prior to infection. The monkeys were infected intramuscularly with 100 x LD₅₀ EMC virus (determined in mice). The control treated monkeys died at 134, 158 and 164 hours post infection. Interferon treatments with 10⁶ units were by the intravenous route at -4, +2, 23, 29, 48, 72, 168 and 240 hours, relative to infection. The bacterial leukocyte interferon was a column chromatography fraction from a lysate of E. coli 294/pLelF A 25 at a specific activity of 7.4 x 10⁶ units/mg protein. The control bacterial proteins were an equivalent column fraction from a lysate of E. coli 294/pBR322 at twice the total protein concentration. The leukocyte interferon standard was Sendai virus induced interferon from normal human "buffy-coat" cells, purified chromatographically to a specific activity of 32 x 10⁶ units/mg protein.

The control monkeys showed progressive lethargy, loss of balance, flaccid paralysis of the hind-limbs and watering of the eyes commencing around 8 hours prior to death. The interferon treated monkeys showed none of these abnormalities; they remained active at all times and developed no viremia. The one monkey in the control group which did not develop viremia by 4 days died latest (164 hours post infection) but showed high titers of virus in the heart and brain on post mortem. The interferon treated monkeys did not develop antibodies to EMC virus as determined 14 and 21 days after infection. These results demonstrate that the antiviral effects of LelF preparations in the infected animals can be attributed solely to interferon because the contaminating proteins are quite different in the bacterial and buffy coat preparations. In addition these results indicate that glycosylation is not required for the in vivo antiviral activity of LelF A.

### J. Isolation of cDNAs for additional mature leukocyte interferons

DNA from the fully characterized LelF A cDNA-containing plasmid was excised with Pst I, isolated electrophoretically, and labelled with ³²p. The resulting radioactively labelled DNA was used as a probe to screen additional E. coli 294 transformants. obtained identically as those in Part C, by the in situ colony screening procedure of Grunstein and Hogness (see above). Colonies were isolated which hybridized in varying amounts to the probe. Plasmid DNA from these colonies and the ten hybridizing colonies referred to in Part G above was isolated by Pst I cutting and characterized by three different methods. First, these Pst fragments were characterized by their restriction endonuclease digestion patterns with the enzymes Bgl II, Pvu II, and Eco RI. This analysis allowed the classification of at least eight different types (LelF A, LelF B, LelF C, LelF D, LelF E, LelF F, LelF G and LelF H), shown in Figure 5, which approximates the location of various restriction cuts relative to the by-now known presequence and coding sequence of LeIF A. One of these, LeIF D, is believed to be identical to that reported by Nagata et al., Nature 284, 316-320 (1980).

Secondly, certain of the DNAs were tested by the hybridization selection assay described by Cleveland et al., Cell 20, 95-105 (1980) for the ability to selectively remove LelF mRNA from poly-A containing KG-1 cell RNA. LeIF A, B, C and F were positive by this assay. Third, the latter Pst fragments were inserted in an expression plasmid, E. coli 294 was transformed with the plasmid, and the fragments were expressed. The expression products, believed to have been pre-interferons, were all positive by CPE assay for interferon activity, albeit marginally active in the case of the LelF F fragment. In addition to the foregoing, all of the LelF types described have been sequenced.

### K. Direct expression of a second mature leukocyte interferon (LeIF B)

The sequence of the isolated fragment comprising the gene for mature LelF B shows the first fourteen nucleotides of types A and B to be identical. Accordingly a fragment from pLelF A25 bearing the trp-promoter-operator, ribosome binding site and the start of the LelF A (= B) gene was isolated and combined with the remaining portion of the B sequence in an expression plasmid. The salient restriction maps for the Pst fragment of pL4 (a plasmid comprising the LelF B Pst-ended gene depicted in Figure 5) and pLelF A25 are shown, respectively, in Figures 7a and 7b.

To obtain the approximately 950 b.p. Sau3a to Pstl fragment from the sequence shown in Figure 7a several steps were necessary because of the presence of one or more intervening Sau3a restriction sites, i.e.:
1. The following fragments were isolated:
   a) 110b b.p. from Sau3a to EcoRI;
   b) 132 b.p. from EcoRI to Xba;
   c) >700 b.p. from Xba to Pst.
2. Fragments (1a) and (1b) were ligated and cut with Xba and BgIII to preclude self-polymerization through Sau 3a and Xba end terminals (the relevant Sau3a site was within a BgIII site; BgIII cuts to leave a Sau3a sticky end). A 242 b.p. fragment was isolated.
3. The product of (2) and (1c) were ligated and cut with Pstl and BgIII, again to prevent self-polymerization. An approximate 950 b.p. fragment, Sau 3a to Pstl of Figure 7a, was isolated. This fragment comprised that portion of the LelF B gene not common to LelF A.
4. An approximate 300 b.p. fragment (HindIII to Sau3a) comprising the trp promoter-operator, ribosome binding site, ATG start signal and cysteine codon of LelF A was isolated from pLelF A25.
5. An approximately 3600 b.p. fragment Pstl to Hindlll was isolated from pBR322. This comprised the replicon and encoded tetracycline but not ampicillin resistance.
6. The fragments obtained in steps 3, 4 and 5 were triple-ligated and the resulting plasmid transformed into E. coli K-12 strain 294.

Transformants were miniscreened (Birnboim et al., Nucleic Acids Res. 7, 1513-1523 [1979]) and plasmid samples were digested with EcoRI. Digests yielded three fragments characteristic of: 1) The EcoRI-EcoRI trp promoter fragment; 2) The internal EcoRI to EcoRI fragment of pL4; and 3) protein translational start signal to EcoRI fragment of pL4.

In CPE assay, bacterial extracts from clones made in the foregoing fashion typically assay at about 10 x 10⁶ units interferon activity per liter at A₅₅₀ = 1. One representative clone prepared in this manner is E. coli 294/pLelF B trp 7.

### L. Direct expression of further mature leukocyte interferons (LeIF C, D, F, H, I and J)

Additional full-length gene fragments that comprise other LelF types may be tailored and placed in expression vehicles for expression as in the case of LelF A. Complete sequencing by conventional means will reveal whether a restriction site lies sufficiently near the first amino acid codon of the mature interferon type as to permit convenient resort to the approach employed in part H, supra, for the expression of mature LelF A, i.e., elimination of the presequence by restriction cutting and replacement of codons for the N-terminal amino acids lost in presequence elimination by ligation of a synthetic DNA fragment. Failing that, the following procedure may be employed. Briefly, this entails cleaving the presequence-containing fragment precisely before the point at which the codon for the first amino acid of the mature polypeptide begins, by:
1. converting the double stranded DNA to single-stranded DNA in a region surrounding that point;
2. hybridizing to the single-stranded region formed in step (a) a complementary primer length of single-stranded DNA, the 5' end of the primer lying opposite the nucleotide adjoining the intended cleavage site;
3. restoring that portion of the second strand eliminated in step 1 which lies in the 3' direction from the primer by reaction with DNA polymerase in the presence of adenine. thymine. guanine and cytosine-containing deoxynucleotide triphosphates; and
4. digesting the remaining single-stranded length of DNA which protrudes beyond the intended cleavage point.

A short length of synthetic DNA terminating, at the 3' end of the coding strand, with the translation start signal ATG can then be ligated by, e.g., blunt-end ligation to the resulting tailored gene for the mature interferons and the gene inserted into an expression plasmid and brought under the control of a promoter and its associated ribosome binding site.

In a manner similar to that employed in part K, supra, gene fragments encoding LelF C and LelF D were appropriately configured for direct bacterial expression. The expression strategy for these additional leukocyte interferons included, in each case, resort to the approximately 300 b.p. fragment (HindIII to Sau3a) comprising the trp promoter-operator, ribosome binding site, ATG start signal and cysteine codon of LelF A from pLelF A25. To this were combined gene fragments from the additional interferon genes encoding their respective amino acid sequences beyond the initial cysteine common to all. Each resulting plasmid was used to transform E. coli K-12 strain 294. Ligations to form the respective genes were as follows:

### LeIF C

Isolate the following fragments from pLelF C:
(a) 35 b.p. from Sau 3a to Sau 96
(b) >900 b.p. Sau 96 to Pst I
(c) Isolate an approximate 300 b.p. fragment (Hind III - Sau 3a) from pLelF A-25 as in part K (4) supra.
(d) Isolate the approximately 3600 b.p. fragment of part K (5) supra.

### Construction:

(1) Ligate (a) and (c). Cleave with BgIII, HindIII and isolate the approximately 335 b.p. product.
(2) Triple ligate (1) + (b) + (d) and transform E. coli with the resulting plasmid.

A representative clone made in this manner is E. coli K-12 strain 294/pLeIF C trp 35.

### LeIF D

Isolate from pLelF D:
a) 35 b.p. from Sau3a to Avall
b) 150 b.p. from Avall to BgIII
c) approx. 700 b.p. from Bglll to Pstl
   Isolate from pLelF A25:
d) 300 b.p. from Hindlll to Sau3a
   Isolate from pBR322:
e) approx. 3600 b.p. from HindIII to PstI

### Construction:

(1) ligate (a) + (b), cut with BgIII and purify a 185 b.p. product (1).
(2) ligate (1) + (d), cut with HindIII, BgIII, and purify the approx. 500 b.p. product (2).
(3) ligate (2) + (c) + (e) and transform E. coli with the resulting plasmid.

A representative clone made in this manner is E. coli K-12 strain 294/pLelF D trp 11.

### LeIF F

The LelF F containing fragment may be tailored for direct expression through reassembly made convenient by the complete homology of amino acids 1-13 of LelF B and LelF F. A trp promoter-containing fragment (a) with appropriately configured ends is obtained from pHGH207, described above, via Pst I and Xba I digestion followed by isolation of the ca. 1050 b.p. fragment. A second fragment (b) is obtained as the larger of the fragments resulting from Pst I and BgIII digestion of the plasmid pHKY 10. Fragment (a) contains approximately half the gene encoding ampicillin resistance; fragment (b) contains the remainder of that gene and the entire gene for tetracycline resistance save for the associated promoter. Fragments (a) and (b) are combined via T4 ligase and the product treated with XbaI and BgIII to eliminate dimerization, forming a fragment (c) comprising the trp promoter-operator and genes for tetracycline and ampicillin resistance.

A fragment (d) of approximately 580 b.p. is obtained by AvaII and BgIII digestion of pLeIF F. This comprises codons for amino acids 14-166 of LelF F.

A fragment (e) (49 b.p.) is obtained by Xbal and Avall digestion of pLelF B. Fragment (e) encodes amino acids 1-13 of LelF F.

Fragments (c), (d) and (e) are triple ligated in the presence of T4 ligase. The cohesive ends of the respective fragments are such that the composite plasmid circularizes correctly, bringing the tetracycline resistance gene under the control of the trp promoter-operator along with the gene for mature LelF F, such that bacteria transformed with the desired plasmid may be selected on tetracycline-containing plates. A representative clone prepared in this manner is E. coli K-12 strain 294/pLelF F trp 1.

### LeIF H

The complete LelF H gene may be configured for expression as a mature leukocyte interferon as follows:
1. Plasmid pLelF H is subjected to Haell and Rsal digestion with isolation of the 816 b.p. fragment extending from the signal peptide amino acid 10 to the 3' noncoding region.
2. The fragment is denatured and subjected to repair synthesis with Klenow fragment of DNA polymerase I (Klenow et al., Proc. Natl. Acad. Sci. U.S.A. 65, 168 [1970]), employing the synthetic deoxyribooligonucleotide primer 5'-dATG TGT AAT CTG TCT.
3. The resulting product is cleaved with Sau3a and a 452 b.p. fragment representing amino acids 1 to 150 isolated.
4. Sau3a and Pstl digestion of pLelF H and isolation of the resulting 500 b.p. fragment yields a gene encoding amino acids 150 through the end of the coding sequence.
5. Fragments isolated in steps (3) and (4) are ligated to form a fragment: 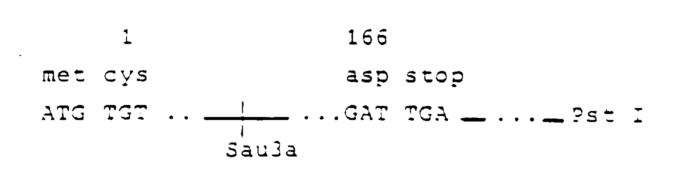
1 166
met cys asp stop
ATG TGT .. ...GAT TGA ... Pst I
Sau3a
 encoding the 166 amino acids of LelF H.
6. pLelF A trp 25 is digested with Xbal, blunt-ended with DNA polymerase I and the product digested with Pst I. The large resulting fragment may be isolated and ligated with the product of step (5) to form an expression plasmid capable, upon transformation of E. coli K-12 strain 294 or other host bacteria, of expressing mature LelF H.

### LeIF I

The particular sequence of LeJF I is not specifically claimed.

The phage λ Charon 4A recombinant library of the human genome constructed by Lawn et al., Cell 15, 1157 (1978), was screened for leukocyte interferon genes by procedures described by Lawn et al. (supra) and Maniatis et al., Cell 15, 687 (1978). A radioactive LelF probe derived from the cDNA clone LelF A, was used to screen approximately 500.000 plaques. Six LelF genome clones were obtained in this screening. Following rescreening and plaque purification, one of these clones, λHLeIF2, was selected for further analysis.

Using the method described above, other probes can be used to advantage to isolate additional LelF clones from the human genome. These, in turn, can be employed to produce additional leukocyte interferon proteins in accordance with this invention.
1. The 2000 b.p. EcoRI fragment of clone λHLeIF2 was subcloned into pBR325 at the EcoRI site. The resulting plasmid LelF I was cleaved with EcoRI and the 2000 b.p. fragment isolated. The deoxyoligonucleotide dAATTCTGCAG (an EcoRI - PstI convertor) was ligated to the 2000 b.p. EcoRI fragment and the resulting product cleaved with Pstl to give a 2000 b.p. fragment containing PstI ends, This was cleaved with Sau96 and a 1100 b.p. fragment isolated which has one Pstl and one Sau96 end.
2. The plasmid pLelF C trp 35 was digested with Pstl and Xbal. The large fragment was isolated.
3. The small Xbal - Pstl fragment from pLelF C trp 35 was digested with Xbal and Sau96. A 40 b.p. Xbal - Sau96 fragment was isolated.
4. The fragments isolated in steps (1), (2) and (3) were ligated to form the expression plasmid pLelF I trp 1.

### LeIF J

The particular sequence of LeJF J is not specifically claimed.
1. The plasmid pLelF J contains a 3.8 kilobase Hindlll fragment of human genomic DNA which includes the LelF J gene sequence. A 760 b.p. Ddel - Rsal fragment was isolated from this plasmid.
2. The plasmid pLelF B trp 7 was cleaved with HindIII and Ddel and a 340 b.p. Hindlll - Ddel fragment isolated.
3. The plasmid pBR322 was cleaved with Pstl, blunt ended by incubation with DNA polymerase I (Klenow fragment), then digested with Hindlll. The large (∼3600 b.p.) fragment was isolated.
4. Fragments isolated in steps (1), (2) and (3) were ligated to form the expression plasmid pLelF J trp 1.

### M. Purification

The content of leukocyte interferon in bacterial extracts may be enhanced by successive:
1. Polyethylene - imine precipitation, in which most of the cellular protein, including the interferon, remains in the supernatant.
2. Ammonium sulfate fractionation, in which interferon comes out of solution in 55% saturated ammonium sulfate.
3. Suspension of the ammonium sulfate pellet in 0.06 M potassium phosphate, 10 mM Tris-HCI, pH 7.2, and dialysis against 25 mM Tris-HCI, pH 7.9 (interferon activity remains in solution).
4. Chromatography of the above supernatant, pH adjusted to 8.5, on a DEAE-cellulose column (eluting with a linear gradient of 0 to 0.2 M NaCI in 25 mM Tris-HCl, pH 8.5).
5. Adsorption on Cibachrome Blue-Agarose or hydroxylapatite and elution with 1.5 M KCI or 0.2 M phosphate solution respectively (optional).
6. Molecular sizing on a Sephadex® G-75 column.
7. Cation exchange chromatography on CM-cellulose in 25 mM ammonium acetate at pH 5.0, developed with an ammonium acetate gradient (to 0.2 M ammonium acetate).

The above process yields material of >95% purity.

The material can also be purified by size exclusion chromatography, reverse phase (RP-8) high pressure liquid chromatography or affinity chromatography on immobilized antiinterferon antibodies.

Alternatively the material from step 4 above can be loaded on a monoclonal antibody column, prepared as described by Milstein, Scientific American 243, 66 (1980), and eluted with 0.2 M acetic acid, 0.1% Triton and 0.15 M NaCI.

In an alternative, preferred embodiment, the leukocyte interferon produced by the procedure described hereinbefore can be purified by the following steps:
1. Frozen cell pellets containing the expressed leukocyte interferon are broken up manually or by appropriate size reduction equipment. The partially thawed cells are suspended in 4 volumes of buffer A, containing 0.1 M Tris adjusted to pH 7.5-8.0, 10% (wiv) sucrose, 0.2 M NaCI, 5 mM EDTA, 0.1 mM PMSF and 10-100 mM MgCl₂. The suspension is held to approximately 4°C.
   The suspension is passed through a homogenizer at about 6000 psi followed by a second pass at less than 1000 psi. Effluent from the homogenizer from both passes is cooled in an ice bath.
2. Polyethylene-imine is added slowly to the homogenate to a concentration of about 0.35% and allowed to stand for about 30 minutes. The solids are removed by centrifugation or filtration. This step is temperature controlled or performed sufficiently quickly that the supernatant (filtrate) is kept at less than 10°C. The supernatant (filtrate) is concentrated by ultrafiltration to approximately 1/10 the original volume. Particulate matter or haziness in the retentate may be removed by an appropriate filter such as a microporous membrane.
3. The clarified solution is loaded directly onto a monoclonal antibody column at a flux of 5-8 cm/hr. (e.g. 25-40 ml/hr. on 2.6 cm diameter column). After loading the column is washed with approximately 10 column volumes of 25 mM Tris-HCI, pH 7.5-8.5, including NaCI (0.5 M) and surfactant such as Triton X-100 (0.2%) or equivalent. Following the wash the column is rinsed with about 10 column volumes of solution containing 0.15 M NaCI and surfactant such as Triton X-100 (0.1%) or equivalent. The column is eluted with 0.2 M acetic acid containing surfactant such as Triton X-100 (0.1%) or equivalent. The protein peak from the monoclonal antibody column (as determined by UV absorbence or other convenient assay) is pooled and the pH adjusted to approximately 4.5 with 1 N NaOH or 1.0 M Tris base.
4. The pooled interferon peak is loaded onto a cationic exchanger such as Whatman CM52 cellulose or equivalent which has been equilibrated with a suitable buffer such as ammonium acetate pH 4.5 (50 mM). After loading, the column is washed with equilibrating buffer until the UV absorbence of the effluent has reached a plateau so that little additional protein is eluting from the column. The column is then eluted with 25 mM ammonium acetatei0.12 M sodium chloride or a combination which optimizes recovery of interferon and affords a lyophilized cake having satisfactory appearance and solubility properties.

The monoclonal antibodies employed in the preferred embodiment described above can be prepared by the procedures described by Staehelin et al., Proc. Natl. Acad. Sci. U.S.A. 78, 1848-52 (1981). Monoclonal antibodies are purified and covalently linked to Affigel-10 as described below:

### Preparation and purification of monoclonal antibodies from ascitic fluid.

Five female Balb/c mice were each inoculated with 5 to 10 x 10⁶ hybridoma cells from mid-log growth phase. About 5 x 10⁶ viable cells obtained from the mouse producing fluid were inoculated intraperitoneally into each of 10 or more mice. The ascitic fluid was collected repeatedly (2 to 4 times) from each mouse. Up to three transfers and collections may be performed from one group of mice to the next. Ascitic fluid from mice at each transfer was pooled.

Cells and debris were removed from the ascitic fluid by low speed centrifugation (500-1000 x g) for 15 minutes. Then centrifugation was performed for 90 minutes at 18,000 rpm. The supernatant was frozen and stored at -20° C. After thawing, additional fibrin and particulate material were removed by centrifugation at 35,000 rpm for 90 minutes. Batches of ascitic fluid from each transfer were tested for specific antibody activity by a solid phase antibody-binding assay (Staehelin et al., supra) and pooled if found satisfactory.

Concentration of protein in the pooled solutions was estimated by the approximation that 1 mg of protein yields an absorbance of 1.2 at 280 nm in a cuvette with a path length of 1.0 cm. Ascites fluids with high levels of antibody contain 30 to 35 mg protein/ml. This is equivalent to 4-7 mg of specific antibody/ml. The fluid was diluted with PBS (0.01 M sodium phosphate, pH 7.3, 0.15 M NaCI) to a protein concentration of 10 to 12 mg/ml.

To each 100 ml of diluted solution, 90 ml of room temperature saturated ammonium sulfate solution was added slowly with vigorous stirring at 0°C. The suspension was kept in ice for 40 to 60 minutes, then centrifuged for 15 minutes at 10,000 rpm at 4°C. The supernatant was decanted and drained well. The protein pellets were dissolved in 0.02 M Tris.HCI (pH 7.9)/0.04 M NaCI (Buffer I).The protein solution was dialyzed for 16 to 18 hours at room temperature against 100 volumes of Buffer I with at least one change of the buffer. The dialyzed solution was centrifuged at 15,000 rpm for 10 minutes to remove undissolved material. About 30-35% of the original amount of total protein in the ascitic fluid was recovered as estimated by absorption at 280 nm.

The solution containing 30-40 mg of protein per ml was then applied to a column of DEAE-cellulose equilibrated with Buffer I. A column bed volume of at least 100 ml was used for each gram of protein applied. The antibody was eluted from the column with a linear NaCI gradient containing 0.02 M Tris.HCI, pH 7.9, from 0.04 M to 0.5 M NaCI. Pooled peak fractions eluting between 0.06 and 0.1 M NaCI were concentrated by precipitation with an equal volume of room temperature saturated ammonium sulfate and centrifugation. The protein pellets were dissolved in 0.2 M NaHCO3 (pH- 8.0)/0.3 M NaCI (Buffer II) followed by dialysis against three changes of the same buffer at room temperature. The dialyzed solutions were centrifuged at 20,000 x g for 15 minutes to remove any insoluble material. Protein concentration was adjusted to 20 to 25 mg/ml with Buffer II.

### Preparation of immunoadsorbants.

Affigel-10 (BioRad Laboratories, Richmond, California) was washed on a sintered glass filter three times with ice-cold isopropanol followed by three washed with ice-cold distilled water. The gel slurry (∼ 50% in cold water) was transferred to plastic tubes and sedimented by a brief centrifugation. The supernatant was aspirated. The packed gel was mixed with an equal volume of purified antibody solution and rotated end-over-end at 4°C for 5 hours. After reaction, the gel was centrifuged, then washed twice with Buffer III (0.1 M NaHCO₃/0.15 M NaCI) to remove uncoupled antibody. Protein determination of the combined washes revealed that more than 90% of antibody was coupled to the gel.

To block unreacted sites. the gel was mixed with an equal volume of 0.1 M ethanoiamine.HCI (pH 8) and rotated end-over-end at room temperature for 60 minutes. The gel slurry was wasned free of reactants with PBS and stored in PBS in the presence of 0.02% (w/v) sodium azide at 4°C.

### N. Parenteral administration

LelF may be parenterally administered to subjects requiring antitumor or antiviral treatment, and to those exhibiting immunosuppressive conditions. Dosage and dose rate may parallel that currently in use in clinical investigations of human derived materials, e.g., about (1-10) x 10⁶ units daily, and in the case of materials of purity greater than 1%, likely up to, e.g., 5 x 10⁷ units daily.

As one example of an appropriate dosage form for essentially homogeneous bacterial LelF in parenteral form, 3 mg LelF of specific activity of, say, 2 x 10⁸ units/mg may be dissolved in 25 ml of 5 N human serum albumin, the solution is passed through a bacteriological filter and the filtered solution aseptically subdivided into 100 vials, each containing 6 x 10⁶ units pure interferon suitable for parenteral administration. The vials are preferably stored in the cold (-20°C) prior to use.

The compounds of the present invention can be formulated according to known methods to prepare pharmaceutically useful compositions, whereby the polypeptide hereof is combined in admixture with a pharmaceutically acceptable carrier vehicle. Suitable vehicles and their formulation are described in Remington's Pharmaceutical Sciences by E.W. Martin, which is hereby incorporated by reference. Such compositions will contain an effective amount of the interferon protein hereof together with a suitable amount of vehicle in order to prepare pharmaceutically acceptable compositions suitable for effective administration to the host. One preferred mode of administration is parenteral.

## Claims

1. A mature human bacterially produced leukocyte interferon characterized in that it consists of 165-166 amino acids and contains Cys-Asp-Leu in position 1, 2 and 3 or it consists of 166 amino acids and contains Cys-Asn-Leu in positions 1, 2 and 3 and such mature leukocyte interferon with at the N-terminus an additional methionine residue.

2. An interferon as claimed in claim 1 characterized in that it consists of 165 amino acids with Cys-Asp-Leu in positions 1, 2 and 3 and Asp in position 114 or of 166 amino acids with Cys-Asp-Leu in positions 1, 2 and 3 and Glu or Val in position 114 or Cys-Asn-Leu in positions 1, 2 and 3 and Glu in position 114.

3. An interferon as claimed in claim 1 characterized in that it consists of 166 amino acids with Met in position 1, Cys-Asp-Leu in positions 2, 3 and 4 and Asp in position 115 or of 167 amino acids with Met in position 1, Cys-Asp-Leu in positions 2, 3 and 4 and Glu or Val in position 115 or Cys-Asn-Leu in positions 2, 3 and 4 and Glu in position 115.

4. An interferon as claimed in claims 1 to 3 containing the partial amino acid sequence

Cys-Ala-Trp-Glu-Val-Val-Arg-Ala-Glu-Ile-Met-Arg-Ser.

5. Mature human leukocyte interferon B(LeJF B) characterized by the amino acid sequence

Cys Asp Leu Pro Gln
Thr His Ser Leu Gly Asn Arg Arg Ala Leu Ile Leu Leu Ala Gln
Met Arg Arg Ile Ser Pro Phe Ser Cys Leu Lys Asp Arg His Asp
Phe Glu Phe Pro Gln Glu Glu Phe Asp Asp Lys Gln Phe Gln Lys
Ala Gln Ala Ile Ser Val Leu His Glu Met Ile Gln Gln Thr Phe
Asn Leu Phe Ser Thr Lys Asp Ser Ser Ala Ala Leu Asp Glu Thr
Leu Leu Asp Glu Phe Tyr Ile Glu Leu Asp Gln Gln Leu Asn Asp
Leu Glu Val Leu Cys Asp Gln Glu Val Gly Val Ile Glu Ser Pro
Leu Met Tyr Glu Asp Ser Ile Leu Ala Val Arg Lys Tyr Phe Gln
Arg Ile Thr Leu Tyr Leu Thr Glu Lys Lys Tyr Ser Ser Cys Ala
Trp Glu Val Val Arg Ala Glu Ile Met Arg Ser Phe Ser Leu Ser
Ile Asn Leu Gln Lys Arg Leu Lys Ser Lys Glu.

6. Mature human leukocyte interferon C(LeJF C)
characterized by the amino acid sequence

Cys Asp Leu Pro Gln
Thr His Ser Leu Gly Asn Arg Arg Ala Leu Ile Leu Leu Gly Gln
Met Gly Arg Ile Ser Pro Phe Ser Cys Leu Lys Asp Arg His Asp
Phe Arg Ile Pro Gln Glu Glu Phe Asp Gly Asn Gln Phe Gln Lys
Ala Gln Ala Ile Ser Val Leu His Glu Met Ile Gln Gln Thr Phe
Asn Leu Phe Ser Thr Glu Asp Ser Ser Ala Ala Trp Glu Gln Ser
Leu Leu Glu Lys Phe Ser Thr Glu Leu Tyr Gln Gln Leu Asn Asp
Leu Glu Ala Cys Val Ile Gln Glu Val Gly Val Glu Glu Thr Pro
Leu Met Asn Glu Asp Ser Ile Leu Ala Val Arg Lys Tyr Phe Gln
Arg Ile Thr Leu Tyr Leu Ile Glu Arg Lys Tyr Ser Pro Cys Ala
Trp Glu Val Val Arg Ala Glu Ile Met Arg Ser Leu Ser Phe Ser
Thr Asn Leu Gln Lys Arg Leu Arg Arg Lys Asp.

7. Mature human leukocyte interferon D (LeIF D) characterized by the amino acid sequence

Cys Asp Leu Pro Glu
Thr His Ser Leu Asp Asn Arg Arg Thr Leu Met Leu Leu Ala Gln
Met Ser Arg Ile Ser Pro Ser Ser Cys Leu Met Asp Arg His Asp
Phe Gly Phe Pro Gln Glu Glu Phe Asp Gly Asn Gln Phe Gln Lys
Ala Pro Ala Ile Ser Val Leu His Glu Leu Ile Gln Gln Ile Phe
Asn Leu Phe Thr Thr Lys Asp Ser Ser Ala Ala Trp Asp Glu Asp
Leu Leu Asp Lys Phe Cys Thr Glu Leu Tyr Gln Gln Leu Asn Asp
Leu Glu Ala Cys Val Met Gln Glu Glu Arg Val Gly Glu Thr Pro
Leu Met Asn Val Asp Ser Ile Leu Ala Val Lys Lys Tyr Phe Arg
Arg Ile Thr Leu Tyr Leu Thr Glu Lys Lys Tyr Ser Pro Cys Ala
Trp Glu Val Val Arg Ala Glu Ile Met Arg Ser Leu Ser Leu Ser
Thr Asn Leu Gln Glu Arg Leu Arg Arg Lys Glu.

8. Mature human leukocyte interferon F (LeIF F) characterized by the amino acid sequence

Cys Asp Leu Pro Gln
Thr His Ser Leu Gly Asn Arg Arg Ala Leu Ile Leu Leu Ala Gln
Met Gly Arg Ile Ser Pro Phe Ser Cys Leu Lys Asp Arg His Asp
Phe Gly Phe Pro Gln Glu Glu Phe Asp Gly Asn Gln Phe Gln Lys
Ala Gln Ala Ile Ser Val Leu His Glu Met Ile Gln Gln Thr Phe
Asn Leu Phe Ser Thr Lys Asp Ser Ser Ala Thr Trp Glu Gln Ser
Leu Leu Glu Lys Phe Ser Thr Glu Leu Asn Gln Gln Leu Asn Asp
Met Glu Ala Cys Val Ile Gln Glu Val Gly Val Glu Glu Thr Pro
Leu Met Asn Val Asp Ser Ile Leu Ala Val Lys Lys Tyr Phe Gln
Arg Ile Thr Leu Tyr Leu Thr Glu Lys Lys Tyr Ser Pro Cys Ala
Trp Glu Val Val Arg Ala Glu Ile Met Arg Ser Phe Ser Leu Ser
Lys Ile Phe Gln Glu Arg Leu Arg Arg Lys Glu.

9. Mature human leukocyte interferon H (LeIF H) characterized by the amino acid sequence

Cys Asn Leu Ser Gln
Thr His Ser Leu Asn Asn Arg Arg Thr Leu Met Leu Met Ala Gln
Met Arg Arg Ile Ser Pro Phe Ser Cys Leu Lys Asp Arg His Asp
Phe Glu Phe Pro Gln Glu Glu Phe Asp Gly Asn Gln Phe Gln Lys
Ala Gln Ala Ile Ser Val Leu His Glu Met Met Gln Gln Thr Phe
Asn Leu Phe Ser Thr Lys Asn Ser Ser Ala Ala Trp Asp Glu Thr
Leu Leu Glu Lys Phe Tyr Ile Glu Leu Phe Gln Gln Met Asn Asp
Leu Glu Ala Cys Val Ile Gln Glu Val Gly Val Glu Glu Thr Pro
Leu Met Asn Glu Asp Ser Ile Leu Ala Val Arg Lys Tyr Phe Gln
Arg Ile Thr Leu Tyr Leu Met Glu Lys Lys Tyr Ser Pro Cys Ala
Trp Glu Val Val Arg Ala Glu Ile Met Arg Ser Phe Ser Phe Ser
Thr Asn Leu Gln Lys Arg Leu Arg Arg Lys Asp.

10. A human leukocyte interferon characterized by an amino acid sequence as claimed in any one of claims 5 to 9 with an additional methionine residue at the amino terminal.

11. A human leukocyte interferon as claimed in any one of claims 1 to 10 bacterially produced.

12. A human leukocyte interferon as claimed in any one of claims 1 to 10 produced by E. coli.

13. A DNA sequence coding for a human leukocyte interferon as claimed in any one of claims 1 to 10.

14. A DNA sequence selected from the group consisting of sequences B, C, D, F and H of Figure 3.

15. A DNA sequence according to claim 13 or 14 operably linked with a DNA sequence capable of effecting expression of a polypeptide according to any one of claims 1 to 10 in a bacterium.

16. A DNA sequence according to claim 13 or 14 operably linked with a DNA sequence capable of effecting expression of a polypeptide according to any one of claims 1 to 10 in E. coli.

17. A replicable expression vehicle capable, in a transformant bacterium, of expressing a polypeptide according to any one of claims 1 to 10.

18. A replicable expression vehicle capable, in a transformant E. coli, of expressing a polypeptide according to any one of claims 1 to 10.

19. A replicable expression vehicle as claimed in claim 17 or claim 18 which is a plasmid.

20. Plasmid pLeIF B trp 7 comprising the E. coli trp promoter and, under the control of said promoter, the nucleotide sequence coding for LeIF B.

21. Plasmid pLeIF F trp 1 comprising the E. coli trp promoter and, under the control of said promoter, the nucleotide sequence coding for LeIF F.

22. Plasmid pLeIF C trp 35 comprising the E. coli trp promoter and, under the control of said promoter, the nucleotide sequence coding for LeIF C.

23. Plasmid pLeIF D trp 11 comprising the E. coli trp promoter and, under the control of said promoter, the nucleotide sequence coding for LeIF D.

24. Plasmid pLeIF H comprising the E. coli trp promoter and, under the control of said promoter, the nucleotide sequence coding for LeIF H.

25. A bacterium transformed with a vehicle as claimed in any one of claims 17 to 24.

26. A strain of E. coli transformed with an expression vehicle as claimed in any one of claims 17 to 24.

27. E. coli K-12 strain 294 transformed with an expression vehicle as claimed in any one of claims 17 to 24.

28. A pharmaceutical composition containing a therapeutically effective amount of a mature human leukocyte interferon as claimed in any one of claims 1 to 10 and a carrier material suitable for pharmaceutical administration.

29. A pharmaceutical composition as claimed in claim 28 for parenteral administration.

30. The use of the compounds claimed in any one of claims 1 to 10 for the preparation of pharmaceutical compositions.

31. The use of the DNA sequences claimed in any one of claims 13 to 16 in the microbial production of a compound claimed in any one of claims 1 to 10.

32. The use of an expression vehicle as claimed in any one of claims 17 to 24 in the bacterial production of a compound claimed in any one of claims 1 to 10.

33. The use of a bacterium as claimed in any one of claims 25 to 27 in the production of a compound claimed in any one of claims 1 to 10.

34. The compounds claimed in any one of claims 1 to 10 whenever used for the treatment of tumors and viral infections or for preparing pharmaceutical compositions useful for such treatment.

35. The DNA sequences claimed in any one of claims 13 to 16 whenever used in the microbial production of a compound claimed in any one of claims 1 to 10.

36. An expression vehicle as claimed in any one of claims 17 to 24 whenever used in the bacterial production of a compound claimed in any one of claims 1 to 10.

37. A bacterium as claimed in any one of claims 25 to 27 whenever used in the production of a compound claimed in any one of claims 1 to 10.

38. A process for preparing a human leukocyte interferon claimed in any one of claims 1 to 10 which process comprises causing a culture of bacterium, transformed with a replicable expression vehicle capable of expressing said polypeptide, to grow up and express said polypeptide and recovering said polypeptide.

39. A process for preparing bacteria capable of expressing a human leukocyte interferon claimed in any one of claims 1 to 10 which process comprises transforming a bacterium with a replicable expression vehicle capable of expressing said polypeptide and cultivating the transformed bacterium.

40. A process for preparing a replicable expression vehicle capable, in a transformant bacterium, of expressing a human leukocyte interferon as claimed in any one of claims 1 to 10 which process comprises constructing a first DNA sequence coding for said polypeptide and operably linking said first DNA sequence with a second DNA sequence capable of effecting bacterial expression of said polypeptide.

## Patentansprüche

1. Ein reifes menschliches, bakteriell hergestelltes Leukozyteninterferon, dadurch gekennzeichnet, daß es aus 165-166 Aminosäuren besteht und Cys-Asp-Leu an den Stellen 1, 2 und 3 enthält, oder daß es aus 166 Aminosäuren besteht und Cys-Asn-Leu an den Stellen 1, 2 und 3 enthält und derartiges reifes Leukozyteninterferon mit einem zusätzlichen Methioninrest am Aminoende.

2. Ein Interferon gemäß Anspruch 1, dadurch gekennzeichnet, daß es aus 165 Aminosäuren mit Cys-Asp-Leu an den Stellen 1, 2 und 3 und Asp an der Stelle 114 oder aus 166 Aminosäuren mit Cys-Asp-Leu an den Stellen 1, 2 und 3 und Glu oder Val an Stelle 114 oder Cys-Asn-Leu an den Stellen 1, 2 und 3 und Glu an der Stelle 114 besteht.

3. Ein Interferon gemäß Anspruch 1, dadurch gekennzeichnet, daß es aus 166 Aminosäuren mit Met an Stelle 1, Cys-Asp-Leu an den Stellen 2, 3 und 4 und Asp an der Stelle 115 oder aus 167 Aminosäuren mit Met an Stelle 1, Cys-Asp-Leu an den Stellen 2, 3 und 4 und Glu oder Val an Stelle 115 oder Cys-Asn-Leu an den Stellen 2, 3 und 4 und Glu an der Stelle 115 besteht.

4. Ein Interferon gemäß einem der Ansprüche 1-3, das die Aminopartialsequenz Cys-Ala-Trp-Glu-Val-Val-Arg-Ala-Glu-Ile-Met-Arg-Ser enthält.

5. Reifes menschliches Leukozyteninterferon B (LeIF B), gekennzeichnet durch die Aminosäuresequenz

Cys Asp Leu Pro Gln
Thr His Ser Leu Gly Asn Arg Arg Ala Leu Ile Leu Leu Ala Gln
Met Arg Arg Ile Ser Pro Phe Ser Cys Leu Lys Asp Arg His Asp
Phe Glu Phe Pro Gln Glu Glu Phe Asp Asp Lys Gln Phe Gln Lys
Ala Gln Ala Ile Ser Val Leu His Glu Met Ile Gln Gln Thr Phe

Asn Leu Phe Ser Thr Lys Asp Ser Ser Ala Ala Leu Asp Glu Thr
Leu Leu Asp Glu Phe Tyr Ile Glu Leu Asp Gln Gln Leu Asn Asp
Leu Glu Val Leu Cys Asp Gln Glu Val Gly Val Ile Glu Ser Pro
Leu Met Tyr Glu Asp Ser Ile Leu Ala Val Arg Lys Tyr Phe Gln
Arg Ile Thr Leu Tyr Leu Thr Glu Lys Lys Tyr Ser Ser Cys Ala
Trp Glu Val Val Arg Ala Glu Ile Met Arg Ser Phe Ser Leu Ser
Ile Asn Leu Gln Lys Arg Leu Lys Ser Lys Glu.

6. Reifes menschliches Leukozyteninterferon C (LeIF C), gekennzeichnet durch die Aminosäuresequenz

Cys Asp Leu Pro Gln
Thr His Ser Leu Gly Asn Arg Arg Ala Leu Ile Leu Leu Gly Gln
Met Gly Arg Ile Ser Pro Phe Ser Cys Leu Lys Asp Arg His Asp
Phe Arg Ile Pro Gln Glu Glu Phe Asp Gly Asn Gln Phe Gln Lys
Ala Gln Ala Ile Ser Val Leu His Glu Met Ile Gln Gln Thr Phe
Asn Leu Phe Ser Thr Glu Asp Ser Ser Ala Ala Trp Glu Gln Ser
Leu Leu Glu Lys Phe Ser Thr Glu Leu Tyr Gln Gln Leu Asn Asp
Leu Glu Ala Cys Val Ile Gln Glu Val Gly Val Glu Glu Thr Pro
Leu Met Asn Glu Asp Ser Ile Leu Ala Val Arg Lys Tyr Phe Gln
Arg Ile Thr Leu Tyr Leu Ile Glu Arg Lys Tyr Ser Pro Cys Ala
Trp Glu Val Val Arg Ala Glu Ile Met Arg Ser Leu Ser Phe Ser
Thr Asn Leu Gln Lys Arg Leu Arg Arg Lys Asp.

7. Reifes menschliches Leukozyteninterferon D (LeIF D), gekennzeichnet durch die Aminosäuresequenz

Cys Asp Leu Pro Glu
Thr His Ser Leu Asp Asn Arg Arg Thr Leu Met Leu Leu Ala Gln
Met Ser Arg Ile Ser Pro Ser Ser Cys Leu Met Asp Arg His Asp
Phe Gly Phe Pro Gln Glu Glu Phe Asp Gly Asn Gln Phe Gln Lys
Ala Pro Ala Ile Ser Val Leu His Glu Leu Ile Gln Gln Ile Phe
Asn Leu Phe Thr Thr Lys Asp Ser Ser Ala Ala Trp Asp Glu Asp
Leu Leu Asp Lys Phe Cys Thr Glu Leu Tyr Gln Gln Leu Asn Asp
Leu Glu Ala Cys Val Met Gln Glu Glu Arg Val Gly Glu Thr Pro
Leu Met Asn Val Asp Ser Ile Leu Ala Val Lys Lys Tyr Phe Arg
Arg Ile Thr Leu Tyr Leu Thr Glu Lys Lys Tyr Ser Pro Cys Ala
Trp Glu Val Val Arg Ala Glu Ile Met Arg Ser Leu Ser Leu Ser
Thr Asn Leu Gln Glu Arg Leu Arg Arg Lys Glu.

8. Reifes menschliches Leukozyteninterferon F (LeIF F), gekennzeichnet durch die Aminosäuresequenz

Cys Asp Leu Pro Gln
Thr His Ser Leu Gly Asn Arg Arg Ala Leu Ile Leu Leu Ala Gln
Met Gly Arg Ile Ser Pro Phe Ser Cys Leu Lys Asp Arg His Asp
Phe Gly Phe Pro Gln Glu Glu Phe Asp Gly Asn Gln Phe Gln Lys
Ala Gln Ala Ile Ser Val Leu His Glu Met Ile Gln Gln Thr Phe
Asn Leu Phe Ser Thr Lys Asp Ser Ser Ala Thr Trp Glu Gln Ser
Leu Leu Glu Lys Phe Ser Thr Glu Leu Asn Gln Gln Leu Asn Asp
Met Glu Ala Cys Val Ile Gln Glu Val Gly Val Glu Glu Thr Pro
Leu Met Asn Val Asp Ser Ile Leu Ala Val Lys Lys Tyr Phe Gln
Arg Ile Thr Leu Tyr Leu Thr Glu Lys Lys Tyr Ser Pro Cys Ala
Trp Glu Val Val Arg Ala Glu Ile Met Arg Ser Phe Ser Leu Ser
Lys Ile Phe Gln Glu Arg Leu Arg Arg Lys Glu.

9. Reifes menschliches Leukozyteninterferon H (LeIF H), gekennzeichnet durch die Aminosäuresequenz

Cys Asn Leu Ser Gln
Thr His Ser Leu Asn Asn Arg Arg Thr Leu Met Leu Met Ala Gln
Met Arg Arg Ile Ser Pro Phe Ser Cys Leu Lys Asp Arg His Asp
Phe Glu Phe Pro Gln Glu Glu Phe Asp Gly Asn Gln Phe Gln Lys
Ala Gln Ala Ile Ser Val Leu His Glu Met Met Gln Gln Thr Phe
Asn Leu Phe Ser Thr Lys Asn Ser Ser Ala Ala Trp Asp Glu Thr
Leu Leu Glu Lys Phe Tyr Ile Glu Leu Phe Gln Gln Met Asn Asp
Leu Glu Ala Cys Val Ile Gln Glu Val Gly Val Glu Glu Thr Pro
Leu Met Asn Glu Asp Ser Ile Leu Ala Val Arg Lys Tyr Phe Gln
Arg Ile Thr Leu Tyr Leu Met Glu Lys Lys Tyr Ser Pro Cys Ala
Trp Glu Val Val Arg Ala Glu Ile Met Arg Ser Phe Ser Phe Ser
Thr Asn Leu Gln Lys Arg Leu Arg Arg Lys Asp.

10. Ein menschliches Leukozyteninterferon gekennzeichnet durch eine Aminosäuresequenz gemäß einem der Ansprüche 5-9 mit einem zusätzlichen Methioninrest am Aminoende.

11. Ein bakteriell hergestelltes menschliches Leukozyteninterferon gemäß einem der Ansprüche 1-10.

12. Ein durch E. coli hergestelltes menschliches Leukozyteninterferon gemäß einem der Ansprüche 1-10.

13. Eine DNA-Sequenz, die für ein menschliches Leukozyteninterferon gemäß einem der Ansprüche 1-10 kodiert.

14. Eine DNA-Sequenz aus der Gruppe der Sequenzen B, C, D, F und H der Figur 3.

15. Eine DNA-Sequenz gemäß Anspruch 13 oder 14, die operabel verbunden ist mit einer DNA-Sequenz, die die Expression eines Polypeptids gemäß einem der Ansprüche 1-10 in einem Bakterium bewirken kann.

16. Eine DNA-Sequenz gemäß Anspruch 13 oder 14, die operabel verbunden ist mit einer DNA-Sequenz, die die Expression eines Polypeptids gemäß einem der Ansprüche 1-10 in E. coli bewirken kann.

17. Ein replikables Expressionsvehikel, das in einem transformierten Bakterium ein Polypeptid gemäß einem der Ansprüche 1-10 zur Expression bringen kann.

18. Ein replikables Expressionsvehikel, das in einem transformierten E. coli Stamm ein Polypeptid gemäß einem der Ansprüche 1-10 zur Expression bringen kann.

19. Ein replikables Expressionsvehikel gemäß einem der Ansprüche 17 oder 18, das ein Plasmid ist.

20. Plasmid pLeIF B trp 7, enthaltend den E. coli trp Promotor und, unter der Kontrolle des besagten Promotors, die Nukleotidsequenz, welche für LeIF B kodiert.

21. Plasmid pLeIF F trp 1, enthaltend den E. coli trp Promotor und, unter der Kontrolle des besagten Promotors, die Nukleotidsequenz, welche für LeIF F kodiert.

22. Plasmid pLeIF C trp 35, enthaltend den E. coli trp Promotor und, unter der Kontrolle des besagten Promotors, die Nukleotidsequenz, welche für LeIF C kodiert.

23. Plasmid pLeIF D trp 11, enthaltend den E. coli trp Promotor und, unter der Kontrolle des besagten Promotors, die Nukleotidsequenz, welche für LeIF D kodiert.

24. Plasmid pLeIF H, enthaltend den E. coli trp Promotor und, unter der Kontrolle des besagten Promotors, die Nukleotidsequenz, welche für LeIF H kodiert.

25. Ein Bakterium, das mit einem Vehikel gemäß einem der Ansprüche 17-24 transformiert ist.

26. Ein E. coli Stamm, der mit einem Expressionsvehikel gemäß einem der Ansprüche 17-24 transformiert ist.

27. E. coli K-12 Stamm 294, transformiert mit einem Expressionsvehikel gemäß einem der Ansprüche 17-24.

28. Eine pharmazeutische Zusammensetzung enthaltend eine therapeutisch wirksame Menge eines reifen menschlichen Leukozyteninterferons gemäß einem der Ansprüche 1-10 und ein für pharmazeutische Verabreichung geeignetes Trägermaterial.

29. Eine pharmazeutische Zusammensetzung gemäß Anspruch 28 für parenterale Verabreichung.

30. Die Verwendung von Verbindungen gemäß einem der Ansprüche 1-10 zur Herstellung von pharmazeutischen Zusammensetzungen.

31. Die Verwendung von DNA-Sequenzen gemäß einem der Ansprüche 13-16 zur mikrobiellen Herstellung einer Verbindung gemäß einem der Ansprüche 1-10.

32. Die Verwendung eines Expressionsvehikels gemäß einem der Ansprüche 17-24 zur bakteriellen Herstellung einer Verbindung gemäß einem der Ansprüche 1-10.

33. Die Verwendung eines Bakteriums gemäß einem der Ansprüche 25-27 zur Herstellung einer Verbindung gemäß einem der Ansprüche 1-10.

34. Die Verbindungen gemäß den Ansprüchen 1-10 zur Verwendung bei der Behandlung von Tumoren und viralen Infektionen und bei der Herstellung pharmazeutischer Zusammensetzungen, die für derartige Behandlungen geeignet sind.

35. Die DNA-Sequenzen gemäß den Ansprüchen 13-16 zur Verwendung bei der mikrobiellen Herstellung einer Verbindung gemäß einem der Ansprüche 1-10.

36. Ein Expressionsvehikel gemäß einem der Ansprüche 17-24 zur Verwendung bei der bakteriellen Herstellung einer Verbindung gemäß einem der Ansprüche 1-10.

37. Ein Bakterium gemäß einem der Ansprüche 25-27 zur Verwendung bei der Herstellung einer Verbindung gemäß einem der Ansprüche 1-10.

38. Ein Verfahren zur Herstellung eines menschlichen Leukozyteninterferons gemäß einem der Ansprüche 1-10, dadurch gekennzeichnet, daß man eine Kultur eines Bakteriums, das mit einem zur Expression eines solchen Polypeptids befähigten replikablen Expressionsvehikel transformiert ist, aufwachsen und besagtes Polypeptid exprimieren läßt und besagtes Polypeptid gewinnt.

39. Ein Verfahren zur Herstellung von Bakterien, die zur Expression eines menschlichen Leukozyteninterferons gemäß einem der Ansprüche 1-10 fähig sind, dadurch gekennzeichnet, daß man ein Bakterium mit einem die Expression besagten Polypeptids bewirkenden, replikablen Expressionsvehikel transformiert und die transformierten Bakterien kultiviert.

40. Ein Verfahren zur Herstellung eines replikablen, in einem transformierten Bakterium die Expression eines menschlichen Leukozyteninterferons gemäß einem der Ansprüche 1-10 bewirkenden Expressionsvehikels, dadurch gekennzeichnet, daß man eine besagtes Polypeptid kodierende erste DNA-Sequenz konstruiert und besagte erste DNA-Sequenz operabel mit einer zweiten, die bakterielle Expression besagten Polypeptids bewirkenden DNA-Sequenz verbindet.

## Revendications

1. Interféron de leucocyte humain mûr produit bactériennement, caractérisé en ce qu'il se composé de 165-166 acides aminés et contient Cys-Asp-Leu en positions 1, 2 et 3 ou qu'il se compose de 166 acides aminés et contient Cys-Asn-Leu en positions 1, 2 et 3 et un tel interféron de leucocyte mûr avec à l'extrémité N un résidu de méthionine additionnel.

2. Interféron suivant la revendication 1, caractérisé en ce qu'il se compose de 165 acides aminés avec Cys-Asp-Leu en positions 1, 2 et 3 et Asp en position 114 ou de 166 acides aminés avec Cys-Asp-Leu en positions 1, 2 et 3 et Glu ou Val en position 114 ou Cys-Asn-Leu en positions 1, 2 et 3 et Glu en position 114.

3. Interféron suivant la revendication 1, caractérisé en ce qu'il se compose de 166 acides aminés avec Met en position 1, Cys-Asp-Leu en positions 2, 3 et 4 et Asp en position 115 ou de 167 acides aminés avec Met en position 1, Cys-Asp-Leu en positions 2, 3 et 4 et Glu ou Val en position 115 ou Cys-Asn-Leu en positions 2, 3 et 4 et Glu en position 115.

4. Interféron suivant l'une quelconque des revendications 1 à 3, contenant la séquence partielle d'acides aminés

Cys-Ala-Trp-Glu-Val-Val-Arg-Ala-Glu-Ile-Met-Arg-Ser.

5. Interféron de leucocyte humain mûr B (LeJF B), caractérisé par la séquence d'acides aminés

Cys Asp Leu Pro Gln
Thr His Ser Leu Gly Asn Arg Arg Ala Leu Ile Leu Leu Ala Gln
Met Arg Arg Ile Ser Pro Phe Ser Cys Leu Lys Asp Arg His Asp
Phe Glu Phe Pro Gln Glu Glu Phe Asp Asp Lys Gln Phe Gln Lys
Ala Gln Ala Ile Ser Val Leu His Glu Met Ile Gln Gln Thr Phe
Asn Leu Phe Ser Thr Lys Asp Ser Ser Ala Ala Leu Asp Glu Thr
Leu Leu Asp Glu Phe Tyr Ile Glu Leu Asp Gln Gln Leu Asn Asp
Leu Glu Val Leu Cys Asp Gln Glu Val Gly Val Ile Glu Ser Pro
Leu Met Tyr Glu Asp Ser Ile Leu Ala Val Arg Lys Tyr Phe Gln
Arg Ile Thr Leu Tyr Leu Thr Glu Lys Lys Tyr Ser Ser Cys Ala
Trp Glu Val Val Arg Ala Glu Ile Met Arg Ser Phe Ser Leu Ser
Ile Asn Leu Gln Lys Arg Leu Lys Ser Lys Glu.

6. Interféron de leucocyte humain mûr C (LeJF C), caractérisé par la séquence d'acides aminés

Cys Asp Leu Pro Gln
Thr His Ser Leu Gly Asn Arg Arg Ala Leu Ile Leu Leu Gly Gln
Met Gly Arg Ile Ser Pro Phe Ser Cys Leu Lys Asp Arg His Asp
Phe Arg Ile Pro Gln Glu Glu Phe Asp Gly Asn Gln Phe Gln Lys
Ala Gln Ala Ile Ser Val Leu His Glu Met Ile Gln Gln Thr Phe
Asn Leu Phe Ser Thr Glu Asp Ser Ser Ala Ala Trp Glu Gln Ser
Leu Leu Glu Lys Phe Ser Thr Glu Leu Tyr Gln Gln Leu Asn Asp
Leu Glu Ala Cys Val Ile Gln Glu Val Gly Val Glu Glu Thr Pro
Leu Met Asn Glu Asp Ser Ile Leu Ala Val Arg Lys Tyr Phe Gln
Arg Ile Thr Leu Tyr Leu Ile Glu Arg Lys Tyr Ser Pro Cys Ala
Trp Glu Val Val Arg Ala Glu Ile Met Arg Ser Leu Ser Phe Ser
Thr Asn Leu Gln Lys Arg Leu Arg Arg Lys Asp.

7. Interféron de leucocyte humain mûr D (LelF D), caractérisé par la séquence d'acides aminés

Cys Asp Leu Pro Glu
Thr His Ser Leu Asp Asn Arg Arg Thr Leu Met Leu Leu Ala Gln
Met Ser Arg Ile Ser Pro Ser Ser Cys Leu Met Asp Arg His Asp
Phe Gly Phe Pro Gln Glu Glu Phe Asp Gly Asn Gln Phe Gln Lys
Ala Pro Ala Ile Ser Val Leu His Glu Leu Ile Gln Gln Ile Phe
Asn Leu Phe Thr Thr Lys Asp Ser Ser Ala Ala Trp Asp Glu Asp
Leu Leu Asp Lys Phe Cys Thr Glu Leu Tyr Gln Gln Leu Asn Asp
Leu Glu Ala Cys Val Met Gln Glu Glu Arg Val Gly Glu Thr Pro
Leu Met Asn Val Asp Ser Ile Leu Ala Val Lys Lys Tyr Phe Arg
Arg Ile Thr Leu Tyr Leu Thr Glu Lys Lys Tyr Ser Pro Cys Ala
Trp Glu Val Val Arg Ala Glu Ile Met Arg Ser Leu Ser Leu Ser
Thr Asn Leu Gln Glu Arg Leu Arg Arg Lys Glu.

8. Interféron de leucocyte humain mûr F (LeIF F), caractérisé par la séquence d'acides aminés

Cys Asp Leu Pro Gln
Thr His Ser Leu Gly Asn Arg Arg Ala Leu Ile Leu Leu Ala Gln
Met Gly Arg Ile Ser Pro Phe Ser Cys Leu Lys Asp Arg His Asp
Phe Gly Phe Pro Gln Glu Glu Phe Asp Gly Asn Gln Phe Gln Lys
Ala Gln Ala Ile Ser Val Leu His Glu Met Ile Gln Gln Thr Phe
Asn Leu Phe Ser Thr Lys Asp Ser Ser Ala Thr Trp Glu Gln Ser
Leu Leu Glu Lys Phe Ser Thr Glu Leu Asn Gln Gln Leu Asn Asp
Met Glu Ala Cys Val Ile Gln Glu Val Gly Val Glu Glu Thr Pro
Leu Met Asn Val Asp Ser Ile Leu Ala Val Lys Lys Tyr Phe Gln
Arg Ile Thr Leu Tyr Leu Thr Glu Lys Lys Tyr Ser Pro Cys Ala
Trp Glu Val Val Arg Ala Glu Ile Met Arg Ser Phe Ser Leu Ser
Lys Ile Phe Gln Glu Arg Leu Arg Arg Lys Glu.

9. Interféron de leucocyte humain mûr H (LelF H), caractérisé par la séquence d'acides aminés

Cys Asn Leu Ser Gln
Thr His Ser Leu Asn Asn Arg Arg Thr Leu Met Leu Met Ala Gln
Met Arg Arg Ile Ser Pro Phe Ser Cys Leu Lys Asp Arg His Asp
Phe Glu Phe Pro Gln Glu Glu Phe Asp Gly Asn Gln Phe Gln Lys
Ala Gln Ala Ile Ser Val Leu His Glu Met Met Gln Gln Thr Phe
Asn Leu Phe Ser Thr Lys Asn Ser Ser Ala Ala Trp Asp Glu Thr
Leu Leu Glu Lys Phe Tyr Ile Glu Leu Phe Gln Gln Met Asn Asp
Leu Glu Ala Cys Val Ile Gln Glu Val Gly Val Glu Glu Thr Pro
Leu Met Asn Glu Asp Ser Ile Leu Ala Val Arg Lys Tyr Phe Gln
Arg Ile Thr Leu Tyr Leu Met Glu Lys Lys Tyr Ser Pro Cys Ala
Trp Glu Val Val Arg Ala Glu Ile Met Arg Ser Phe Ser Phe Ser
Thr Asn Leu Gln Lys Arg Leu Arg Arg Lys Asp.

10. Interféron de leucocyte humain, caractérisé par une séquence d'acides aminés suivant l'une quelconque des revendications 5 à 9 avec un résidu de méthionine additionnel à l'amino terminal.

11. Interféron de leucocyte humain suivant l'une quelconque des revendications 1 à 10, produit bactériennement.

12. Interféron de leucocyte humain suivant l'une quelconque des revendications 1 à 10, produit par E. coli.

13. Séquence d'ADN codant pour un interféron de leucocyte humain suivant l'une quelconque des revendications 1 à 10.

14. Séquence d'ADN choisie dans le groupe comprenant les séquences B, C, D, F et H de la Fig. 3.

15. Séquence d'ADN suivant l'une ou l'autre des revendications 13 et 14, liée activement à une séquence d'ADN pouvant effectuer l'expression d'un polypeptide suivant l'une quelconque des revendications 1 à 10 dans une bactérie.

16. Séquence d'ADN suivant l'une ou l'autre des revendications 13 et 14, liée activement à une séquence d'ADN pouvant effectuer l'expression d'un polypeptide suivant l'une quelconque des revendications 1 à 10 chez E. coli.

17. Véhicule d'expression réplicable pouvant, dans une bactérie transformante, exprimer un polypeptide suivant l'une quelconque des revendications 1 à 10.

18. Véhicule d'expression réplicable pouvant, dans une E. coli transformante, exprimer un polypeptide suivant l'une quelconque des revendications 1 à 10.

19. Véhicule d'expression réplicable suivant l'une ou l'autre des revendications 17 et 18, qui est un plasmide.

20. Plasmide pLelF B trp 7 comprenant le promoteur trp d'E. coli et, sous le contrôle dudit promoteur, la séquence nucléotidique codant pour LelF B.

21. Plasmide pLelF F trp 1 comprenant le promoteur trp d'E. coli et, sous le contrôle dudit promoteur, la séquence nucléotidique codant pour LelF F.

22. Plasmide pLelF C trp 35 comprenant le promoteur trp d'E. coli et, sous le contrôle dudit promoteur, la séquence nucléotidique codant pour LelF C.

23. Plasmide pLelF D trp 11 comprenant le promoteur trp d'E. coli et, sous le contrôle dudit promoteur, la séquence nucléotidique codant pour LelF D.

24. Plasmide pLelH comprenant le promoteur trp d'E. coli et, sous le contrôle dudit promoteur, la séquence nucléotidique codant pour LeIF H

25. Bactérie transformée avec un véhicule suivant l'une quelconque des revendications 17 à 24.

26. Souche d'E. coli transformée avec un véhicule d'expression suivant l'une quelconque des revendications 17 à 24.

27. Souche 294 d'E. coli K-12 transformée avec un véhicule d'expression suivant l'une quelconque des revendications 17 à 24.

28. Composition pharmaceutique contenant une quantité thérapeutiquement efficace d'un interféron de leucocyte humain mûr suivant l'une quelconque des revendications 1 à 10 et une matière formant support appropriée pour l'administration pharmaceutique.

29. Composition pharmaceutique suivant la revendication 28, pour l'administration parentérale.

30. Utilisation des composés suivant l'une quelconque des revendications 1 à 10, pour la préparation de compositions pharmaceutiques.

31. Utilisation des séquences d'ADN suivant l'une quelconque des revendications 13 à 16, dans la production microbienne d'un composé suivant l'une quelconque des revendications 1 à 10.

32. Utilisation d'un véhicule d'expression suivant l'une quelconque des revendications 17 à 24, dans la production bactérienne d'un composé suivant l'une quelconque des revendications 1 à 10.

33. Utilisation d'une bactérie suivant l'une quelconque des revendications 25 à 27, dans la production d'un composé suivant l'une quelconque des revendications 1 à 10.

34. Composés suivant l'une quelconque des revendications 1 à 10, lorsque utilisés pour le traitement de tumeurs et d'infections virales et pour la préparation de compositions pharmaceutiques intéressantes pour un tel traitement.

35. Séquences d'ADN suivant l'une quelconque des revendications 13 à 16, lorsque utilisées dans la production microbienne d'un composé suivant l'une quelconque des revendications 1 à 10.

36. Véhicule d'expression suivant l'une quelconque des revendications 17 à 24, lorsque utilisé dans la production bactérienne d'un composé suivant l'une quelconque des revendications 1 à 10.

37. Bactérie suivant l'une quelconque des revendications 25 à 27, lorsque utilisée dans la production d'un composé suivant l'une quelconque des revendications 1 à 10.

38. Procédé de préparation d'un interféron de leucocyte humain suivant l'une quelconque des revendications 1 à 10, lequel procédé consiste à amener une culture de bactérie, transformée avec un véhicule d'expression réplicable pouvant exprimer le polypeptide précité, à croître et à exprimer ledit polypeptide et à récupérer le polypeptide précité.

39. Procédé de préparation de bactéries pouvant exprimer un interféron de leucocyte humain suivant l'une quelconque des revendications 1 à 10, lequel procédé comprend la transformation d'une bactérie avec un véhicule d'expression réplicable pouvant exprimer le polypeptide précité et la culture de la bactérie transformée.

40. Procédé de préparation d'un véhicule d'expression réplicable pouvant, dans une bactérie transformante, exprimer un interféron de leucocyte humain suivant l'une quelconque des revendications 1 à 10, lequel procédé comprend la construction d'une première séquence d'ADN codant pour le polypeptide précité et la liaison active de ladite première séquence d'ADN avec une seconde séquence d'ADN pouvant effectuer l'expression bactérienne dudit polypeptide.
